Europäisches Patentamt

European Patent Office

Office européen des brevets

(11) Publication number: **0 327 180**
**A2**

(12) **EUROPEAN PATENT APPLICATION**

(21) Application number: 89200230.4

(22) Date of filing: 02.02.89

(51) Int. Cl.⁴: **A61K 39/21** , **C07K 15/04** , **C12N 15/00**

(30) Priority: 03.02.88 US 151976

(43) Date of publication of application:
**09.08.89 Bulletin 89/32**

(84) Designated Contracting States:
**AT BE CH DE ES FR GB GR IT LI LU NL SE**

(71) Applicant: **MicroGeneSys, Inc.**
**400 Frontage Road**
**West Haven Connecticut 06437(US)**

(72) Inventor: **Smith, Gale E.**
**125 Michael Drive**
**Guilford Connecticut 06437(US)**
Inventor: **Cochran, Mark A.**
**415 Brooksvale Avenue**
**Hamden, Connecticut 06518(US)**
Inventor: **Volvovitz, Franklin**
**123 York Street**
**New Haven, Connecticut 06511(US)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

(54) Vaccine containing polypeptides derived from the envelope gene of human immunodeficiency virus type 1.

(57) An Acquired Immunodeficiency Syndrome (AIDS) vaccine containing the Human Immunodeficiency Virus, Type-1 (HIV-1) envelope proteins is produced from cloned HIV-1 envelope genes in a baculovirus-inset cell vector system. The recombinant HIV-1 proteins are purified, assembled into particles and then adsorbed on an aluminum phosphate adjuvant. The resulting adsorbed recombinant HIV-1 virus envelope protein formulation is highly immunogenic in animals and elicits antibodies which bind to the HIV-1 virus envelope and neutralize the infectivity of the virus in in vitro tests.

# VACCINE CONTAINING POLYPEPTIDES DERIVED FROM THE ENVELOPE GENE OF HUMAN IM-MUNODEFICIENCY VIRUS TYPE 1

## BACKGROUND OF THE INVENTION

The Human Immunodeficiency Virus Type-1 (HIV-1) is a retrovirus which causes a systemic infection with a major pathology in the immune system and is the etiological agent (Barre-Sinoussi et al. 1983; Popovic et al. 1984) responsible for Acquired Immunodeficiency Syndrome (AIDS). Clinical isolates of HIV-1 have also been referred to Lymphadenopathy-Associated Virus (Feorino et al. 1984), Human T-cell Leukemia Virus III (Popovic et al. 1984) and AIDS-related Virus (Levy, et al. 1984).

AIDS has become pandemic and the development of a vaccine has become a major priority for world public health. A high percentage of persons infected with HIV-1 show a progressive loss of immune function due to the depletion of T4 lymphocytes. These T4 cells, as well as certain nerve cells, have a molecule on their surface called CD4. HIV-1 recognizes the CD4 molecule through a receptor located in the envelope of the virus particles enters these cells, and eventually replicates and kills the cell. An effective AIDS vaccine might be expected to elicit antibodies which would bind to the envelope of HIV-1 and prevent it from infecting T4 lymphocytes or other susceptible cells.

Vaccines are generally given to healthy individuals before they are exposed to a disease organism as an immune prophylactic. However, it is also reasonable to consider using an effective AIDS vaccine in post-exposure immunization as immunotherapy against the disease (Salk. 1987).

It is widely believed that the HIV-1 envelope is the most promising candidate in the development of an AIDS vaccine (Francis and Petricciani. 1985; Vogt and Hirsh. 1986; Fauci. 1986). The HIV-1 envelope protein is initially synthesized as a 160,000 molecular weight glycoprotein (gp160). The gp160 precursor is then cleaved into a 120,000 molecular weight external glycoprotein (gp120) and a 41,000 molecular weight transmembrane glycoprotein (gp41). These envelope proteins are the major target antigens for antibodies in AIDS patients (Barin, et al. 1985). The native HIV-1 gp120 has been shown to be immunogenic and capable of inducing neutralizing antibodies in rodents, goats, rhesus monkeys and chimpanzees (Robey, et al. 1986).

Due to the very low levels of native HIV-1 envelope protein in infected cells and the risks associated with preparing an AIDS vaccine from HIV-1 infected cells, recombinant DNA methods have been employed to produce HIV-1 envelope antigens for use as AIDS vaccines. Recombinant DNA technology appears to prevent the best option for the production of an AIDS subunit vaccine because of the ability to produce large quantities of safe and economical immunogens. The HIV-1 envelope has been expressed in genetically altered vaccinia virus recombinants (Chakrabarty et al. 1986; Hu et al. 1986; Kieny et al. 1986), bacterial cells (Putney et al. 1986), mammalian cells (Lasky. et al. 1986), and in insect cells. Synthetic peptides derived from amino acids sequences in an HIV-1 gp41 have also been considered as candidate AIDS vaccines (Kennedy. et al. 1986).

The use of a baculovirus-insect cell vector system to produce recombinant HIV-1 envelope proteins is disclosed in copending, coassigned U.S patent application Serial No. 920,197 filed October 16, 1986. The above-identified patent application and the publications referenced herein are herein incorporated and made part of this disclosure.

This system has been demonstrated to be of general utility in producing HIV-1 proteins and other proteins. As examples, the baculovirus Autographa californica nuclear polyhedrosis virus (AcNPV) has been used as a vector for the expression of the full length gp160 and various portions of the HIV-1 envelope gene in infected Spodoptera frugiperda (fall armyworm) cells (Sf9 cells). Also disclosed in the above-identified patent application is the truncated gp160 gene (recombinant number Ac3046), the protein produced from recombinant Ac3046, and a purification technique for the Ac3046 gene product that includes lentil lectin affinity chromatography followed, by gel filtration chromatography. The gp160 protein purified in this manner and aggregated to form particles was found to be highly immunogenic in rodent and primate species.

The ideal AIDS vaccine, in addition to the requirements of being substantially biologically pure and non-pyrogenic, should provide life-long protection against infection with HIV-1 after a single or a few injections. This is usually the case with live attenuated vaccines. When killed bacteria or viruses, or materials isolated from them, such as toxoids or proteins, are used to make a vaccine, there often results a poor antibody response and only short term immunity. To overcome these defi ciencies in a vaccine, an additional component, called an adjuvant, is added which has the property of being able to help stimulate the immune response. An adjuvant in common use in human vaccines (Bomford, 1985) are gels of salts of aluminum (aluminum phosphate or aluminum hydroxide) and is usually referred to as an alum adjuvant.

## SUMMARY OF THE INVENTION

It has been discovered that recombinant HIV-1 gp160 protein, especially when adsorbed onto an adjuvant, such as alum, e.g. aluminum phosphate, is particularly useful as an AIDS vaccine. One aspect of this invention is an AcNPV expression vector having the coding sequence for a portion of the HIV-1 env gene which encompasses the amino acids 1-757 found in the recombinant clone number 3046 and the production of that recombinant HIV-1 envelope protein in insect cells codes for by the amino acid sequences 1-757.

Other aspects of this invention comprise formation of recombinant envelope protein particles from the gene product from the baculovirus recombinant virus that produces the 3046 protein and adsorption of the 3046 particles to aggregates of aluminum phosphate.

## DETAILED DESCRIPTION OF THE INVENTION

The recombinant baculovirus Autographa californica nuclear polyhedrosis virus (AcNPV) which contains the truncated HIV-1 gp160 gene coding for the HIV envelope protein 1-757 amino acids (recombinant Ac3046) is described in copending, coassigned U.S. application Serial No. 920,197, as well as the cloning step employed to construct the recombinant baculovirus containing genes or portions of genes from HIV-1. The following is a detailed description of the genetic engineering steps used to construct Ac3046 expression vector.

In the following description, the materials employed, including enzymes and immunological reagents, were obtained from commercial sources.

Details of the practices of this invention are set forth hereinbelow with reference to the accompanying drawings wherein:

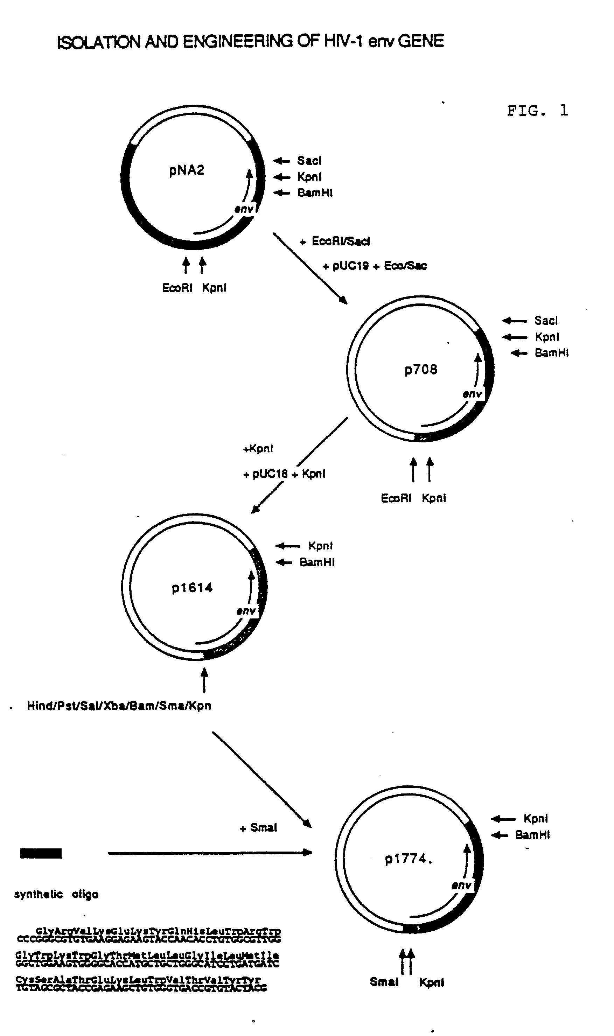

Fig. 1 illustrates the cloning strategy used to isolate the HIV-1 envelope gene (env) from the E. coli plasmid pNA2. The hatched regions are HIV-1 DNA sequences and the open regions are from the cloning vectors. The black region in p1774 is constructed from synthetic oligonucleotides and was introduced as a SmaI-KpnI fragment into the SmaI-KpnI sites of p1614. The sequence of this synthetic oligonucleotide is shown.

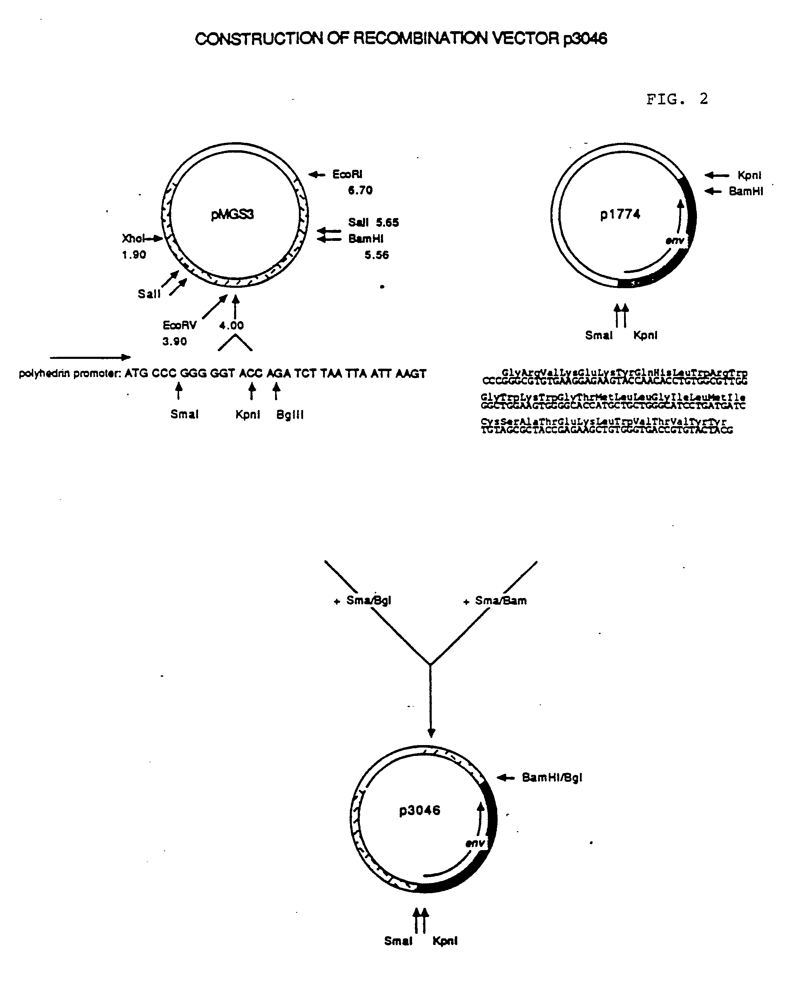

Fig. 2 illustrates the strategy used to construct the recombinant vector (p3046) used to construct the baculovirus expression vector Ac3046. The plasmid pMGS3 contains sequences (cross-hatched areas) from the baculovirus AcNPV on either side of a cloning site at position 4.00. This site has the unique restriction endonuclease sites for SmaI, KpnI, and BglII. In the 5' direction from the 4.00 position is the AcNPV polyhedrin promoter and in the 3' direction is the sequence 5'-TAATTAATTAA-3' which has a translational codon in all three reading frames. The plasmid p1774 and the sequence of the synthetic oligonucleotide region is as described in Fig. 1. The plasmid p3046 contains all of pMGS3 except for the sequence between the SmaI and BglII sites and the HIV-1 envelope gene in p1774.

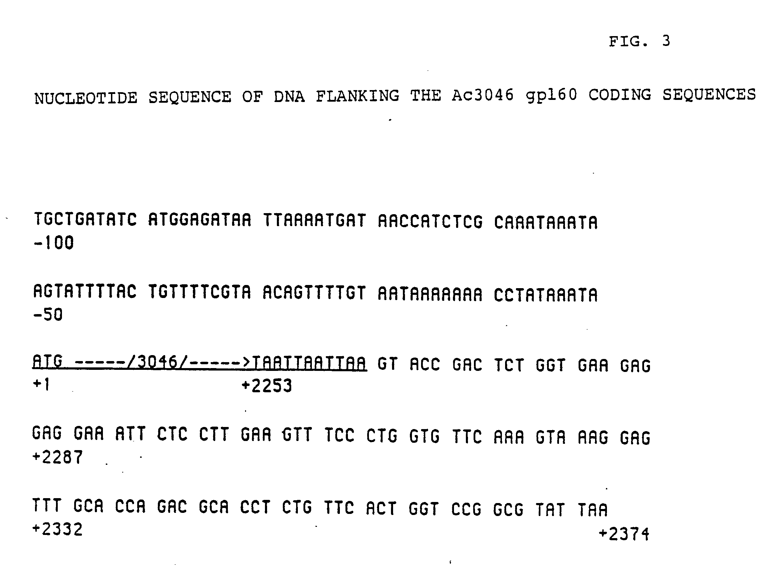

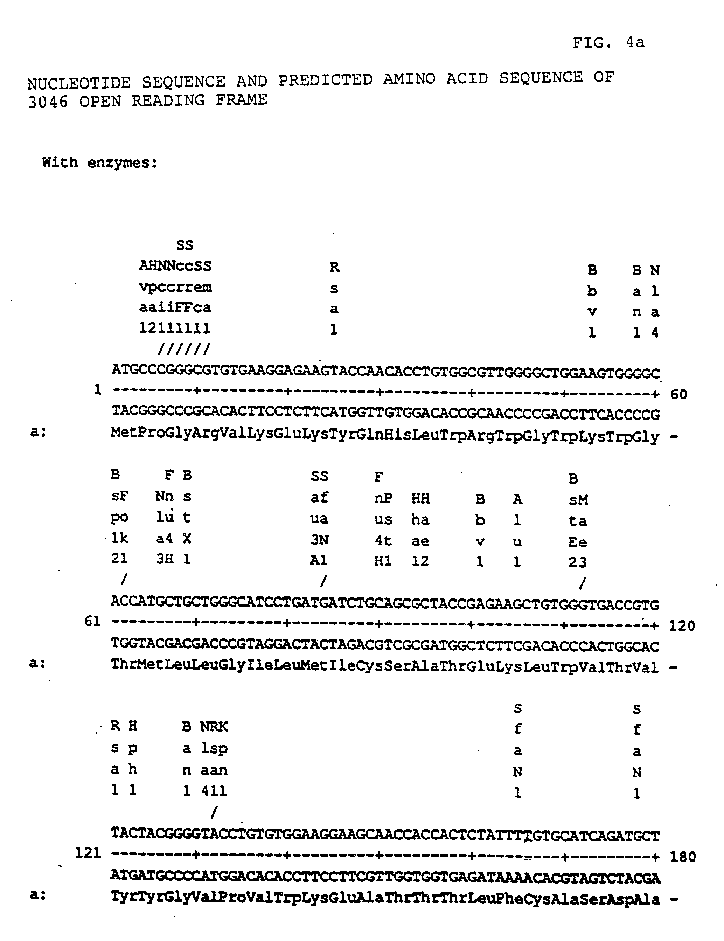

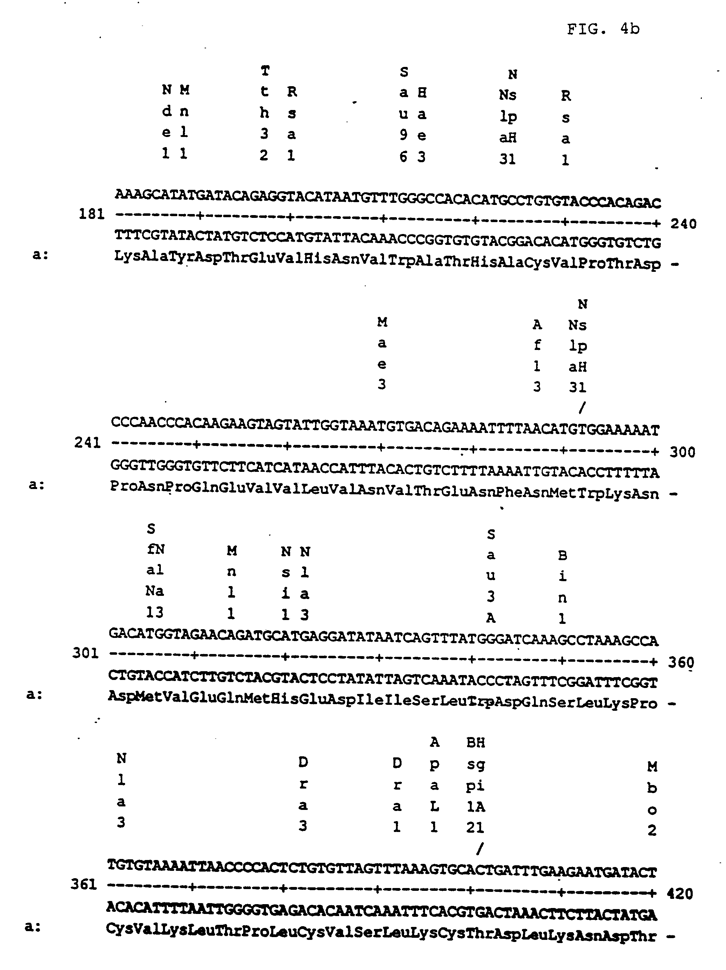

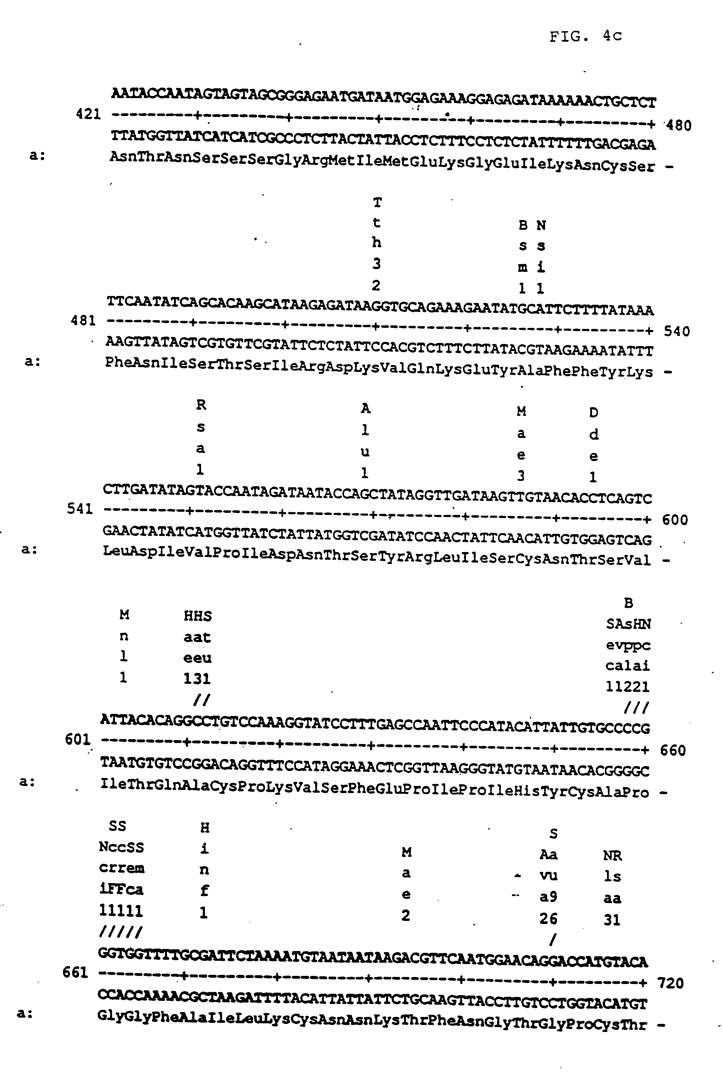

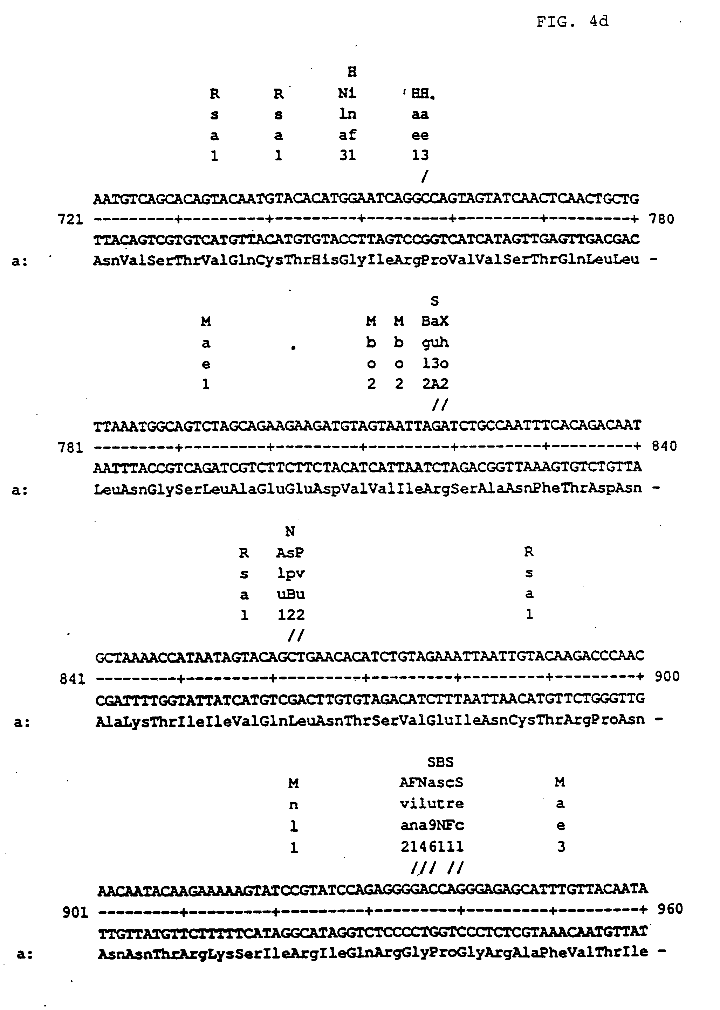

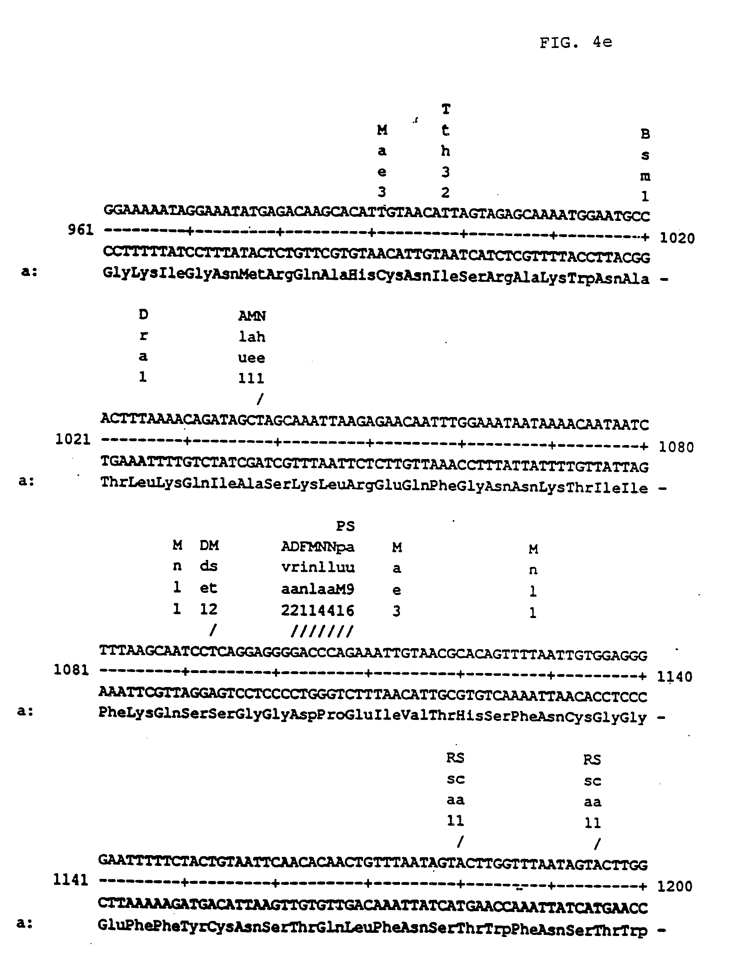

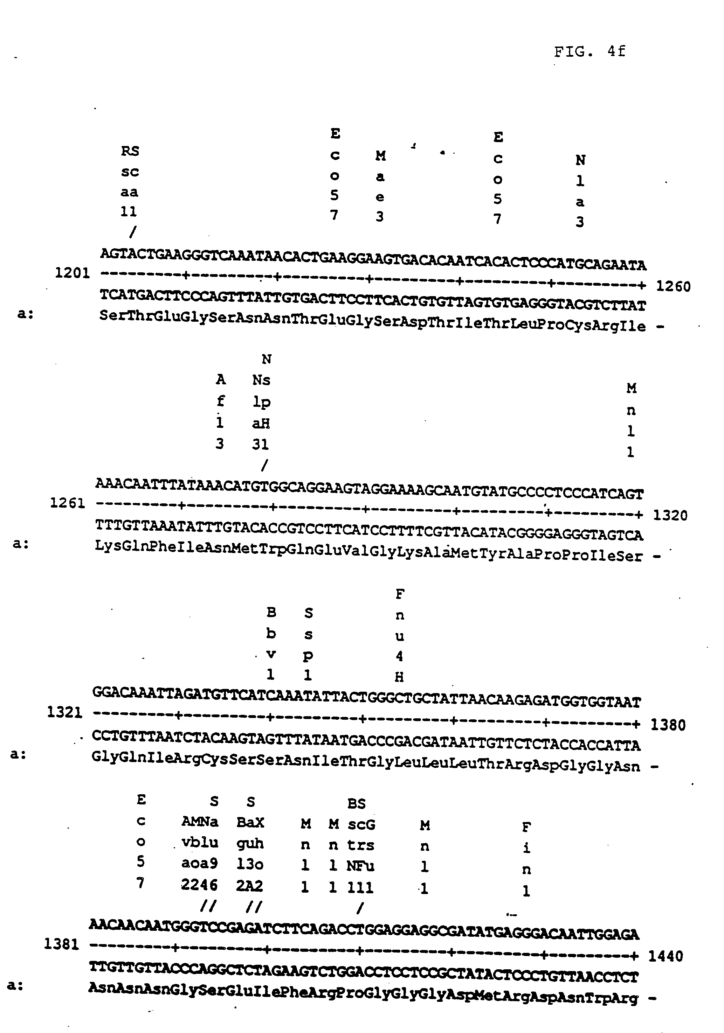

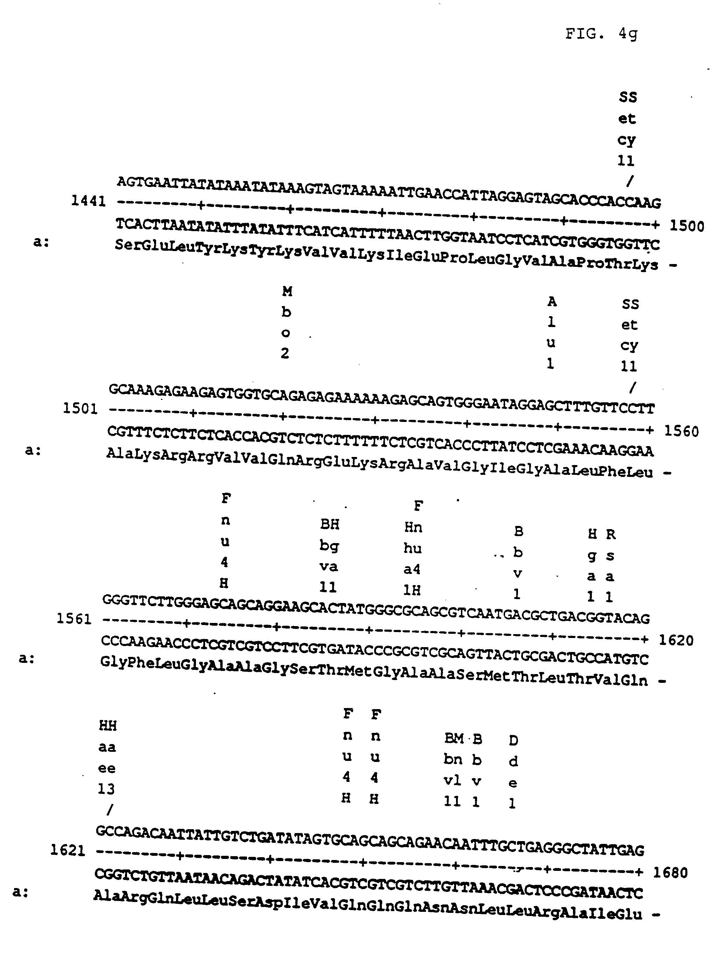

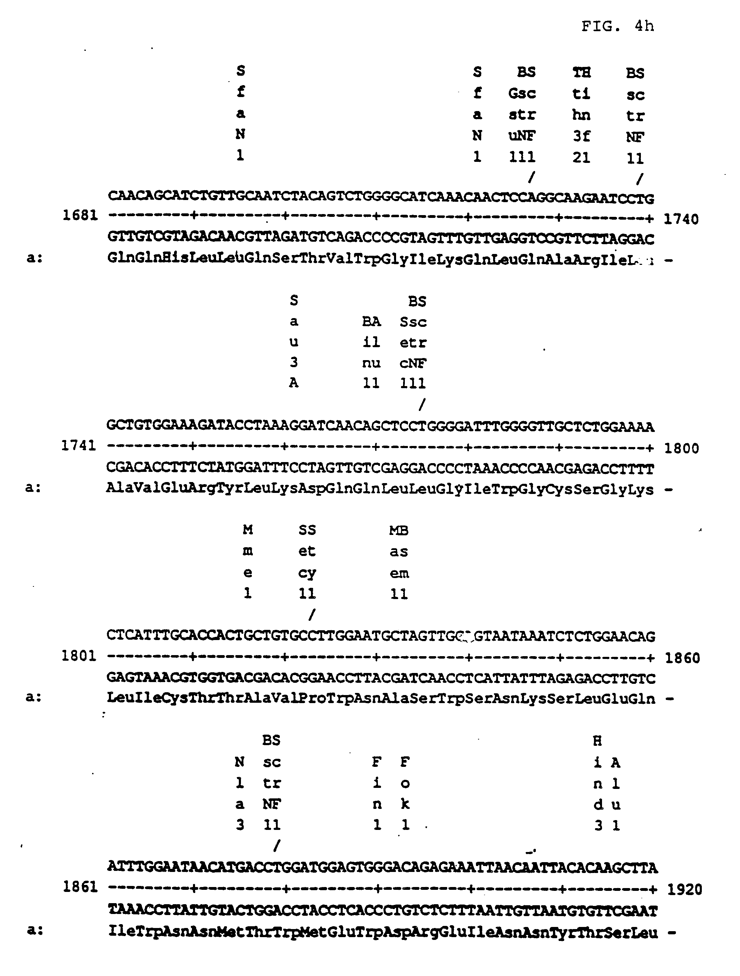

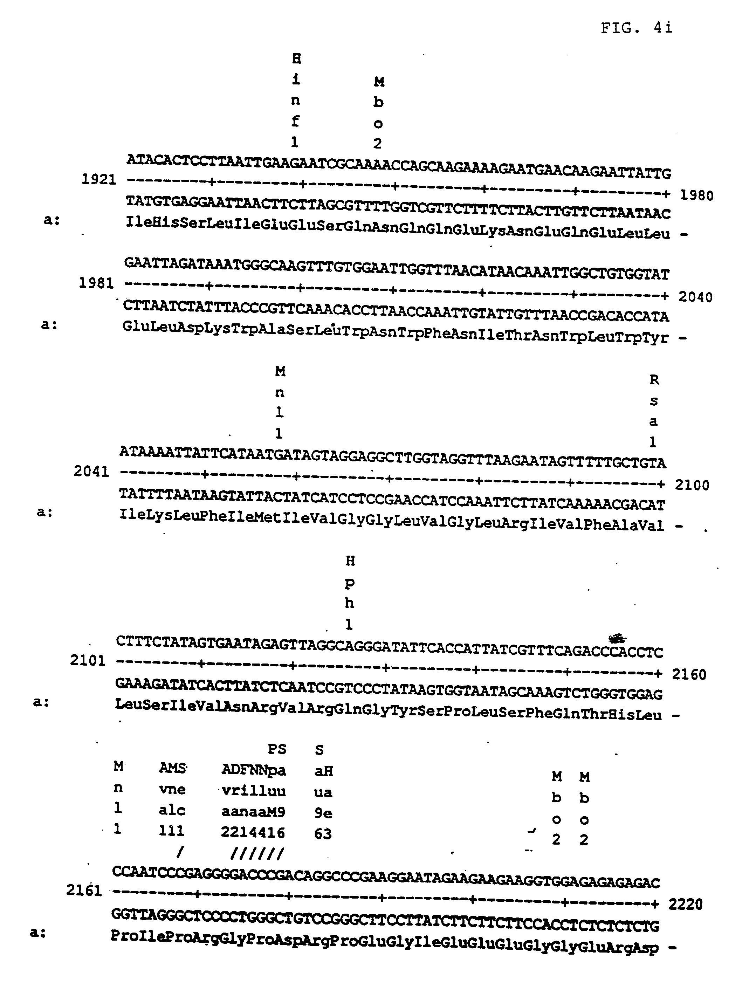

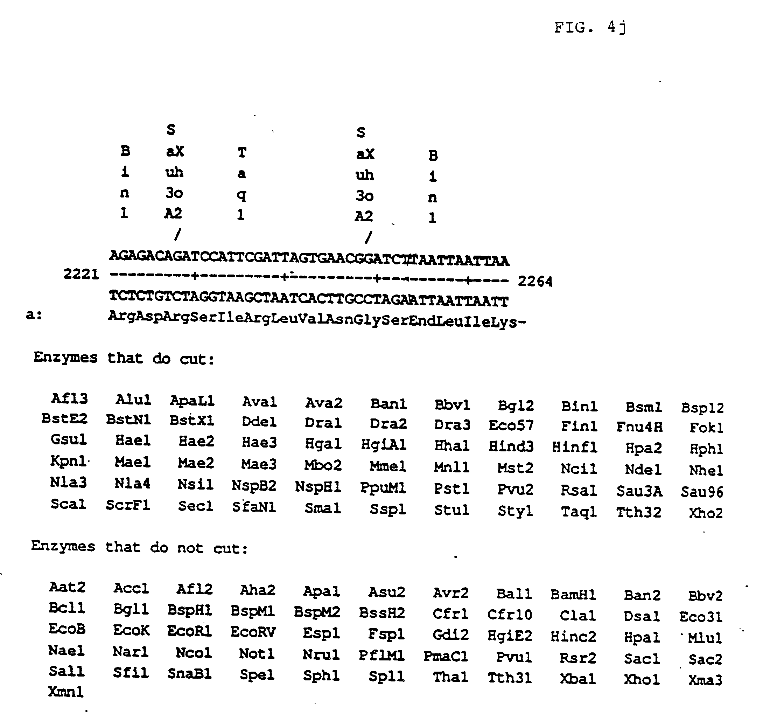

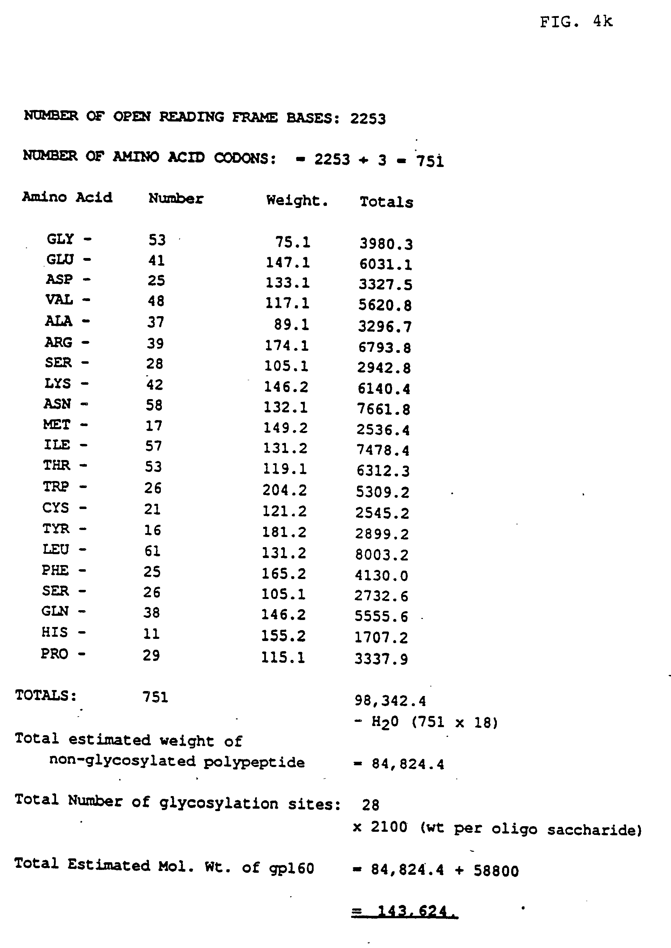

Fig. 3 shows the nucleotide sequences of the DNA flanking the Ac3046 gp160 coding sequences . The 3046 env DNA sequence between +1 and +2264 is shown in Fig. 4.

Figs. 4a-4k show the actual DNA sequence of the HIV-1 env gene segment along with the synthetic oligonucleotide sequences at the 5' end of the env gene in Ac3046 between +1 to +2264. The location of restriction endonuclease sites are listed above the DNA·sequence and the predicted amino acid sequence is listed below the DNA sequence. The bases are numbered on the right and on the left.

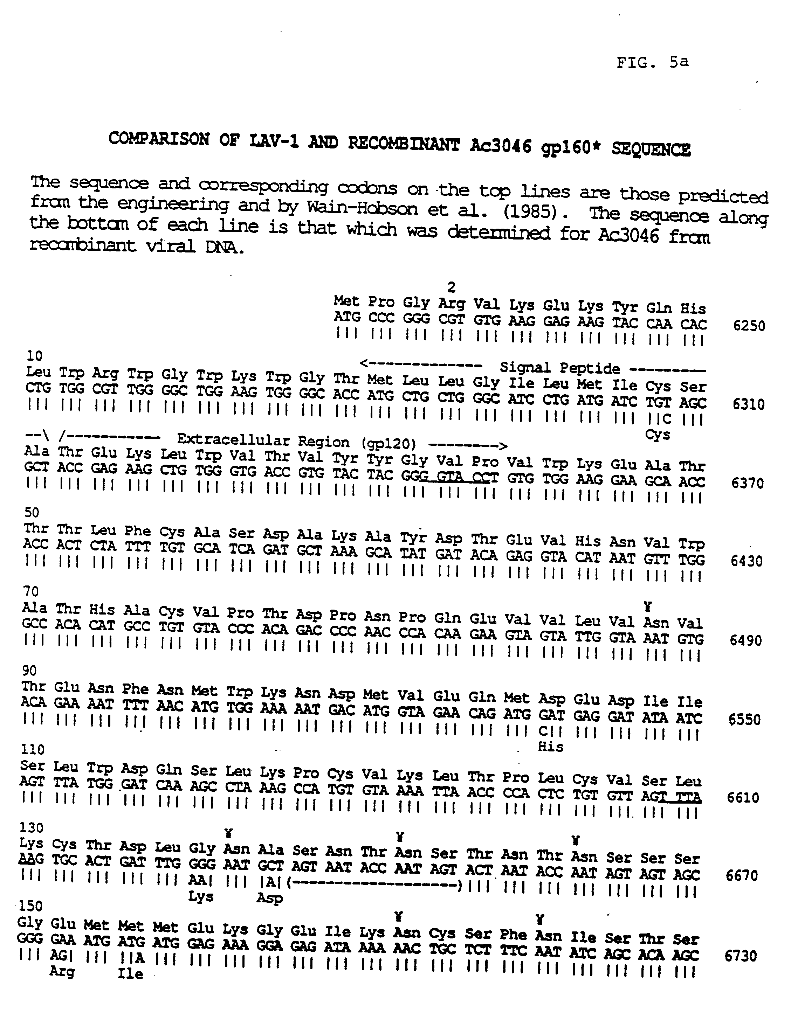

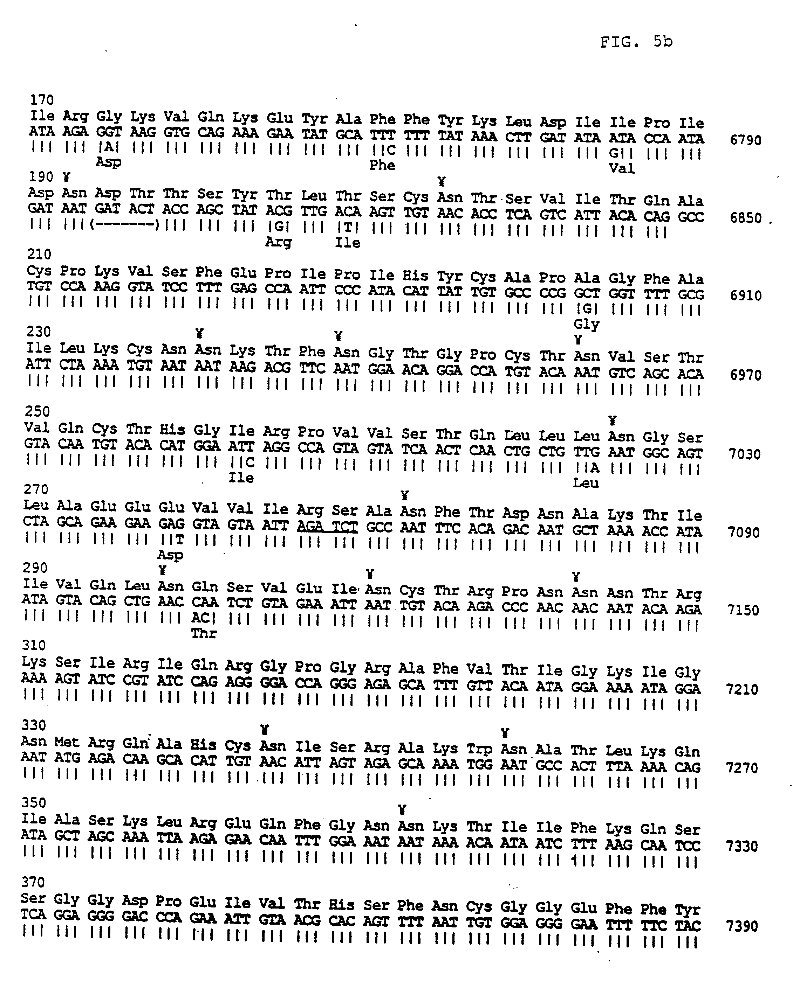

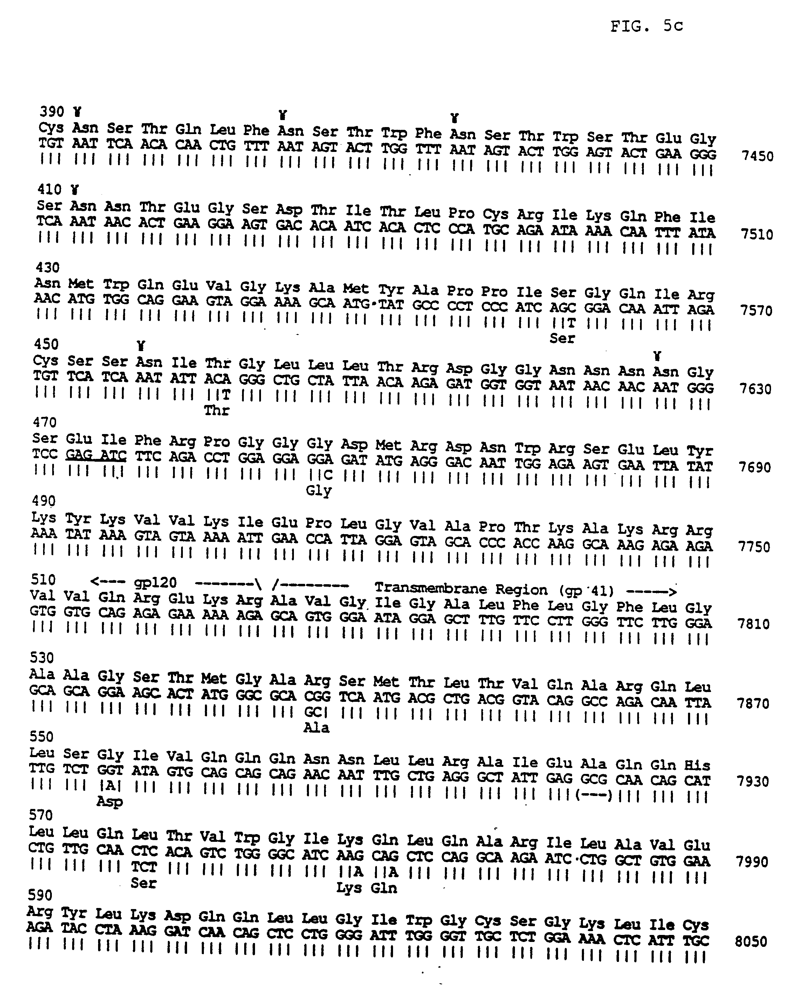

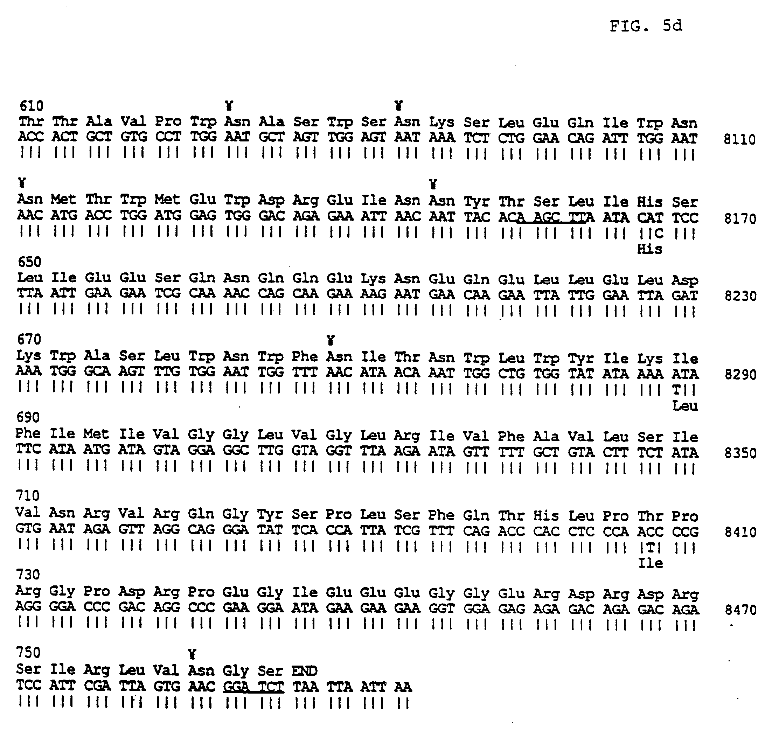

Fig. 5a-5d compare the DNA sequences of the env gene from Ac3046 with a published env gene sequence from LAV-1. The LAV-1 sequence is on the top and Ac3046 is on the bottom. A line ( ) )below the LAV-1 sequence indicates that the sequence in Ac3046 is the same in this position. The DNA sequence numbering used is that desribed by Wain-Hobson. et al 1985 for LAV-1.

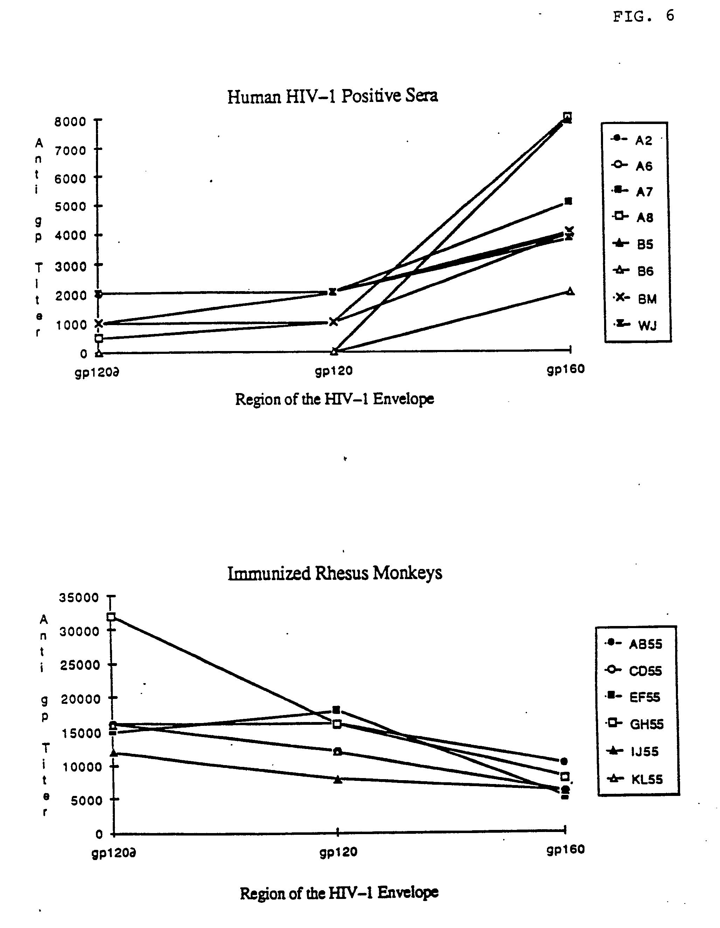

Fig. 6 shows the ELISA end point dilution titers of human HIV-1 antibody positive sera (top graph) and rhesus monkey sera (bottom graph) from animals immunized with gp160 (IJ55, KL55) or gp120 (AB55, CD55, GH55). The ELISA titers were measured against highly purified gp120 and gp160 proteins. The specifically bound antibody was measured with a goat anti-human IgG HRP conjugate. The highest dilution of serum that gives a positive response in the test is the titer.

The following are examples illustrative of the practices of this invention:

## EXAMPLE 1

Construction of the baculovirus recombinant Ac3046 bearing the HIV-1 coding sequence for amino acids 1-757

Cloning and expression of foreign protein coding sequences in a baculovirus vector require that the coding sequence be aligned with the polyhedrin promoter and upstream sequences and on the other side with truncated polyhedrin coding sequences such that homologous recombination with the baculovirus genome results in transfer of the foreign coding sequence aligned with the polyhedrin promoter and an inactive polyhdrin gene.

Accordingly, a variety of insertion vectors were designed for use in AIDS env gene constructions. The insertion vector MGS-3, described below, was designed to supply the ATG translational initiating codon. Insertion of foreign sequences into this vector must be engineered such that the translational frame established by the initiating codon is maintained correctly through the foreign sequences.

The insertion vector MGS-3 was constructed from an EcoRI-I restriction fragment clone of DNA isolated from a plaque purified AcMNPV isolate (WT-1), and was designed to consist of the following structural features: 4000 bp of sequence upstream from the ATG initiating codon of the polyhedrin gene; a polylinker introduced by site directed mutagenesis, which consists of an ATG initiating codon at the exact position of the corresponding polyhedrin codon, and restriction sites SmaI, KpnI, BglI and a universal stop codon segment; 1700 bp of sequence extending from the KpnI restriction site (which is internal to the poloyhedrin gene) through to the terminal EcoRI restriction site of the EcoRI-I clone.

## EXAMPLE 2

Construction of baculovirus recombinants bearing LAV env coding sequences

A recombinant plasmid designated NA-2 consisted of a 21.8 kb segment of an entire AIDS provirus inserted into pUC18. This clone was reportedly infectious since it could produce virus following transfection of certain human cells (Adachi et al. 1986). The complete envelope gene sequences contained in NA-2 were derived from the LAV strain of HIV (Barre-Sinoussi et al. 1983).

The HIV-1 envelope gene was isolated and engineered as described below, see also Fig. 1. The envelope gene was initially isolated from NA-2 as a 3846 bp Ecol/SacI restriction fragment and cloned into the EcoRI/SacI restriction site pUC19. The resultant plasmid was designated as p708. The envelope gene was subsequently reisolated as a 2800 bp KpnI restriction fragment and cloned into the KpnI restriction site of pUC18. The resulting clone was designated p1614. This KpnI restriction fragement contained a slightly truncated piece of the envelope gene such that 121 bp of the N-terminal corresponding sequence was missing. This missing part in the gene, which included the signal peptide sequences, was replaced by insertion of a double-stranded synthetic oligomer which was designed from the LAV amino acid sequence using preferred polyhedrin gene codon usage. To facilitate further manipulation, a new SmaI restriction sequence was concomitantly introduced in place of the ATG initiating codon. The ATG initiation codon will be supplied by the insertion vector. The resultant plasmid was designated a p1774.

Referring now to Fig. 2, restriction fragments from p1774 containing coding sequences of vaious domains of the AIDS envelope were cloned into the MGS vectors such that the ATG initiating codon of the insertion vector was in-frame with the condons of the envelope gene. Construct p3046 consisted of the SmaI/BamHI restriction fragment isolated from p1447 inserted into the SmaI/BglII site of the plasmid vector pMGS-3. This clone contains sequences coding for amino acids 1 through 757 of gp160 and uses a termination codon supplied by the MGS-3 vector.

## EXAMPLE 3

Preparation and Selection of Recombinant Baculovirus

The HIV env gene recombination plasmid p3046 was calcium phosphate pecipitated with AcMNPV DNA (WT-1) and added to uninfected Spodoptera frugiperda cells. The chimeric gene was then inserted into the AcMNPV genome by homologous recombination. Recombinant viruses identified were by an occlusion negative plaque morphology. Such plaques exhibit an identifiable cytopathic effect but no nuclear occlusions. Two additional successive plaque purifications were carried out to obtain pure recombinant virus. Recombinant viral-DNA was analyzed for site-specific insertion of the HIV env sequences by comparing their restrictions and hybridization characteristics to wild-type viral DNA.

## EXAMPLE 4

Expression of HIV env from recombinant baculoviruses in infected insert cells

Expression of HIV-env sequences from the recombinant viruses in insect cells should result in the synthesis of primary translational product in the form of a pre-pro-protein containing all the amino acids coded for from the ATG initiating codon of the expression vector downstream from the polyhedrin promoter. This primary product will consist of amino acids translated from the codons supplied by the recombination vector. The primary translation product of Ac3046 should read Met-Pro-Gly-Arg-Val at the terminus where Arg (position 4) is the Arg at position 2 in the original LAV clone. The Met-Pro-Gly codons are supplied as a result of the cloning strategy.

## EXAMPLE 5

Nucleotide sequence of the gp160 insert and flanking DNA

The nucleotide sequence of the gp160 insert and flanking DNA was determined from restriction fragments isolated from viral expression vector Ac3046 DNA. The sequencing strategy involved the following steps. The 3.9 kbp EcoRV-BamHI fragment was purified by restriction digestion of Ac3046 viral DNA. The Ac3046 vial DNA had been prepared from extracellular virus present in the media of cells being used for a production lot of vaccine.

As shown in Fig. 2, the 3.9 kbp EcoRV-BamHI fragment consists of the entire gp160 gene and 100 bp of upstream and about 1000 bp of downstream flanking DNA. Of this, the nucleotide sequence of the entire gp160 gene was determined, including 100 bp of upstream and 100 bp of downstream flanking DNA.

Briefly, the results of the sequencing revealed a chimeric construct as predicted from the cloning strategy. The sequence of the gp160 was essentially as reported by Wain-Hobson et al. (1985). The sequence of 2253 bases between the presumed translation initiation and termination codons predicts 751 amino acid condons and 28 potential N-linked glycosylation sites. The estimated molecular weight of gp160, including the sugar residues, is approximately 145,000.

Sequence analysis of 200 bases of flanking DNA indicated correct insertion as indicated in Figs, 3, 4 and 5.

## EXAMPLE 6

Amino Acid Sequence of gp160

Using standard automated Edman degradation and HPLC procedures, the N-terminal sequence of the first 15 residues of gp160 was determined to be identical to that predicted from the DNA sequence. The N-terminal methionine is not present on the gp160 protein. This is consistent with the observation that AcNPV polyhedrin protein is also produced without an N-terminal methionine. A summary of the actual 5' gp160 DNA and N-terminal protein sequences, as has been determined by analysis of the AcNPV 3046 DNA and purified gp160, is as follows:

LAV env gene in the AcNPV 3046 expression vector

```
Residue #  2   3   4   5   6   7   8   9   10  11  12  13  14
     -     Pro Gly Arg Val Lys Glu Lys Tyr Gln His Leu Trp Arg

           Trp Gly

           ATG CCC GGG CGT GTG AAG GAG AAG TAC CAA CAC CTG TGG

           CGT TGG GGC
```

Lav env gene in the original LAV-1 clone

```
Residue #  1   2   3   4   5   6   7   8   9   10  11  12  13  14
     Met Arg Val Lys Glu Lys Tyr Gln His Leu Trp Arg Trp

     Gly

     ATG AGA GTG AAG GAG AAG TAT CAG CAC TTG TGG AGA TGG

     GGG
```

## EXAMPLE 7

### Purifications of recombinant gp160

One aspect of the prevent invention is the procedure used to extract and purify the recombinant HIV-1 envelope protein coded for in the Ac3046 expression vector. The recombinant HIV-1 envelope protein gp160 is produced in S. frugiperda cells during the 4-5 days after infection withe Ac3046. Purification of the gp160 protein involves the steps:

1. Washing the Cells
2. Cell Lysis
3. Lentil Lectin Affinity Chromatography
4. Gel Filtration Chromatography
5. Dialysis

The following steps describe the purification of the recombinant gp 160 from Ac3046 infected cells obtained from 2 x 109 infected cells:

### Washing the cells.

Infected cells are washed in a buffer containing 50 mM Tris buffer, pH 7.5, 1 mM EDTA and 1% Triton x-100. The cells are resuspended in this buffer, homogenized using standard methods, and centrifuged at 5000 rpm for 20 min. This process is repeated 3 times.

### Cell Lysis.

After the washed cells are lysed by sonication in 50 mM Tris buffer, pH 8.0-8.5, 4% deoxycholate, 1% B-mercaptoethanol. Sonication is done using standard methods. After sonication, only remnants of the nuclear membrane are intact and these are removed by the centrifugation of 5000 rpm for 30 min. The supernatant containing the extracted gp160 contains no intact cells as determined by light microscopy observations.

6

Gel filtration.

Gel filtration is done in a Pharmacia 5.0 x 50 cm glass column packed with a Sephacryl resin (Pharmacia). The total bed volume is about 1750 ml. To depyrogenate and sanitize the column and tubing connections, at least 6 liters of 0.1 N NaOH is run through the column over a period of 24 hours. The effluent from the column is connected to a UV flow cell and monitor and chart recorder (Pharmacia) then equilibrated with 4 liters of Gel Filtration Buffer. The crude gp 160 is loaded onto the column then developed with Gel Filtration Buffer. The column separates the crude mixture into three major UV absorbing fractions. The first peak comes off between about 500 and 700 mls, the second between 700 and 1400 mls and the third between 1400 and 1900 mls buffer. This same profile is observed on small analytical columns from which it has been determined that the first peak is material that has a molecular weight of $\geq 2,000,000$. This peak is translucent due to a concentration of high molecular weight lipids and lipid complexes. This peak also contains from 10% to 20% of the gp 160 extracted from the infected cells. Apparently this fraction of gp160 is complexed to itself or other cell components to form high molecular weight aggregates. The second broad peak contains the majority of the gp160 and proteins with molecular weights of between about 18,000 and 200,000. The third peak contains little protein and the majority of the UV absorption is due to the B-mercaptoethanol in the sample. When the second peak is first detected from the tracing of the UV absorbance, the effluent from the column is directly onto the lentil lectin column. Once the second peak has come off the column, the effluent is disconnected from the lentil lectin column and directed to waste.

Lentil lectin.

The lentil lectin affinity gel media (Lentil Lectin-Sepharose 4B) was purchased in bulk from Pharmacia. The lentil lectin was isolated by affinity chromatography on Sephadex to greater than 98% purity then immobilized by coupling to Sepharose 4B using cyanogen bromide. The matrix contains about 2 mg ligand per ml of gel. The lentil lectin column is a 5.0 x 30 cm glass column (Pharmacia) containing 125 ml lentil lectin-Sepharose 4B gel. The affinity matrix is reused after being thoroughly washed and regenerated by a procedure recommended by the supplier. When not in use, the gel is stored in the column in a solution of 0.9% NaCl, 1 mM MnCl2, 1 mM CaCl2, and 0.01% thimerasol. The column is washed and equilibrated with 250 ml lentil lectin buffer described above before each use. The crude gp160 is applied to the column directly as it is eluting from the gel filtration column as described above. Once the crude gp160 is bound to the column, it is washed with 800 ml lentil lectin buffer containing 0.1% deoxycholate. Under these conditions all of the gp160 binds to the column. lentil lectin buffer plus 0.3M alpha-methyl mannoside is used to elute the bound glycoproteins which is monitored through a UV monitor at a wavelength of 280 nm.

Purification summary.

Summary of the purification of gp 160 from 1 liter of infected cells:

| Purification Step | Total Protein[1] mg | gp160 Protein mg | % gp160 Total | Contaminants Removed |
|---|---|---|---|---|
| Cell pellet | 1-2000 | 20 | 1-2 | Culture medium |
| 1,2,3rd Wash | 250 | 15 | 6 | Serum Albumin, most Nucleic Acids, and Soluble Cell Proteins |
| Gel Filtration | 120 | 12 | 10 | Lipids, Nucleic Acids, and high mol wt aggregates |
| Lentil Lectin | 14 | 10 | 70 | Nonglycosylated proteins |
| Dialysis | 13 | 9 | 70 | Sugar, deoxycholate, excess Tris buffer |

[1] The total protein content was estimated from the absorbance at 280 nm

**EXAMPLE 8**

Assembly of gp160 Particles.

As one aspect of the present invention, it has been discovered that the gp160 antigen can be assembled into particles of ≥ 2,000,000 molecular weight during purification. The gp160 protein is extracted from the cell as a mixture of 80-90% monomeric (160,000 molecular weight) and 10-20% polymeric (particle form). The gel filtration step removes the aggregated forms of gp160. Attempts to purify the gp160 from this fraction (first peak off the gel filtration column) suggest that it is complexed with other cell proteins, possibly even with membrane fragments. However, the gp160 antigen in the second peak off the gel filtration column has a molecular weight of about 160,000-300,00 and is, therefore, in predominantly monomeric or dimeric form. The formation of aggregates of polymers of gp 160 occurs during the development of the lentil lectin column. It has been determined that the antigen forms aggregates whether it is eluted from the lectin column in 0.5% doxycholate, which is about the 0.2% critical micelle concentration (CMI) for doxycholate, or whether the gp160 is eluted from the column in 0.1% deoxycholate. The size of the aggregates are measured on a high resolution FPLC Superose 12 column (Pharmacia). Samples from representative lots of purified gp160 have a size that is predominantly equal to or greater than the 2,000,000 molecular weight of a blue dextran size standard. It is likely that, as non-glycosylated proteins are removed from association with the gp160 antigen during the binding and washing to the lentil lectin column, the hydrophobic portions of gp160 begin to form intermolecular associations. The deoxycholate is probably not bound to the gp160 as the concentration can be kept above the CMI and the antigen will still form complexes. The assembly of this antigen into aggregates appears to be an intrinsic property of this protein once it is purified. It is possible that the very hydrophobic N-terminal sequence that is present on the gp160 protein contributes to the natural ability of this protein to form particles. After purification, the gp160 complexes can be sterile filtered through an 0.2 micron cellulose acetate filter without significant loss of protein.

Analysis of Particle Formation.

An analysis of purified gp160 by electron microscopy demonstrates that there were protein-like, spherical particles of 30-100 nM. As an additional test for the presence of particles, purified gp160 was analyzed by gel filtration. About 100 μg gp160 was applied to a Superose 12, FPLC gel filtration HR 10/30 column (Pharmacia, Inc.). This column was first calibrated with protein molecular weight standards. The protein profile from this column is highly reproducible with respect to the elution volume being inversely proportional to the molecular weight of protein standards. The column separates the monomeric gp160 from the polymeric forms and excludes globular proteins of ≥ 2 x 106 molecular weight. When developed on this column, essentially all of the purified gp160 elutes in the void volume and is, therefore, ≥ 2 x 106 molecular weight in size.

**EXAMPLE 9**

Adsorption of gp160 to Alum.

The effectiveness of insoluble aluminum compounds as immunologic adjuvants depends on the completeness of adsorption of the antigens on the solid phase. As a part of the present disclosure it was discovered that alum could be made that would efficiently adsorb the gp160 but at a pH that would not reduce the potency of the gp160-alum complex as an immunogen. The factors controlled during the formation of alum (aluminum phosphate gel) are:

1. The optimal pH for adsorption of antigens to alum is about 5.0. However, it was discovered that the gp160 lost immunogenicity at a pH of 6.5 in comparison to a pH of 7.5 so the Alum is made at a pH of 7.1 ± 0.1. It was discovered that essentially 100% of the gp160 will still adsorb to the alum at this pH.

2. The ionic strength from the NaCl present is relatively low and is less than 0.15 M.

3. There is a molar excess of aluminum chloride relative to sodium phosphate to assure that there is an absence of free phosphate ions in the supernatant.

4. The gp160 antigen is added to freshly formed alum to stop crystal growth and minimize the size of the particles.

The procedure to make 100 ml alum and adsorb purified gp160 to the alum so that the final concentration of antigen is 40 $\mu$g/ml is outlined hereinbelow:

Preparation of Reagents (200 mL total formulated lot).

1. Prepare the following solutions in 100 mL sterile, pyrogen-free bottles or beakers. Mix the salts for Solution 1 and Solution 2 and the sodium hydroxide and filter through 0.2$\mu$ cellulose acetate filters into 100 mL sterile, pyrogen-free bottles.

| Solution 1 | AlCl3.6H2O | 0.895 grams |
| | NaHAc.3H2O | 0.136 grams |
| dissolve in 40 mL WFI water, 0.2$\mu$ filter | | |
| Solution 2 | Na3PO.12H2O | 1.234 grams |
| dissolve in 40 mL WFI water, 0.2$\mu$ filter | | |
| Solution 3 | NaOH | 2.0 grams |
| dissolve in 100 mL WFI water, 0.2$\mu$ filter | | |
| Solution 4 | Tris | 1.25 grams |
| dissolve in 100 mL WFI water, add 1 mL to 90 mL WFI, adjust pH to 7.5 with 0.5N HCl, and bring to 100 mL with WFI | | |

2. Autoclave solutions for 30 min; slow exhaust. Cool to room temperature.

Formation of Alum

1. Add Solution 1 (aluminum chloride-sodium acetate to the formulation vessel using 25 mL sterile, disposable pipets. Note the volume of Solution 1 and begin stirring the solution.

2. Add Solution 2 (sodium phosphate) to the vessel using 25 mL sterile, disposable pipets and continue stirring as the precipitate forms and note the volume of Solution 2.

3. Add 3mL Solution 3 (sodium hydroxide) and continue stirring for 5 min. Take an 0.5 ml sample and measure the pH. If the pH is less than 7.0, add an additional 0.5 ml sodium hydroxide, stir for another 5 minutes and measure the pH again. Continue until the pH is between 7.0 and 7.2.

4. Determine the total volume added to the formulation vessel (Solution 1 + Solution 2 + Solution 3), then add sterile WFI to bring the volume to 100 mL.

5. Immediately add 8,000 $\mu$g purified gp160 in 100mL of 1mM Tris pH 7.5 directly into the formulation vessel.

6. Continue stirring for a minimum of 20 minutes, then dispense the formulated vaccine into a sterile vial.

## EXAMPLE 10

Immunogenicity of Alum adsorbed gp160.

An accepted method to determine the immunogenicity of an antigen preparation (vaccine) is to measure the specific antibody response in groups of mice which have been given a single dose of antigen. At the end of 4 weeks the mice are bled and the serum antibody levels to a specified antigen (usually the antigen used to immunize the animal with) measured by a standard antibody test such as an ELISA (enzyme linked immunoabsorbant assay).

The immunogenicity in mice of purified gp160 with no adjuvant at pH 6.0 and pH 7.5 adsorbed as described in Example 9 with alum, or mixed with Freunds Complete Adjuvant are summarized hereinbelow:

| Group | | gp160 | | Seroconversion | |
|---|---|---|---|---|---|
| gp160 | Adjuvant | Lot# | Mean ELISA OD[1] | % | (P/N)[2] |
| 1μg | None, pH 7.5 | 8702 | 0.140 | 57% | 4/6 |
| | None, pH 6.0 | 8702 | 0.110 | 26% | 2/7 |
| | Alum | 8702 | 1.000 | 90% | 9/10 |
| | Alum | 8705 | 2.285 | 100% | 6/6 |
| | Freund's | 8604 | 1.108 | 83% | 5/6 |
| | Freund's | 8702 | 1.396 | 100% | 7/7 |
| 0.1μg | Freund's | 8604 | 0.434 | 67% | 4/6 |
| | | 8705 | 1.003 | 67% | 4/6 |

[1] The mice were bled 28 days post immunization and the sera tested at 1:10 dilution in an ELISA assay against gel-purified gp160. Sim ilar results were obtained using a commercial ELISA (Genetic Systems Inc.; EIA[tm] ELISA) assay against the native HIV-1 proteins at a serum dilution of 1:400.

[2] The number of seroconverted mice (P) to the total number tested (N).

Mice immunized with single 1.0 μg dose of gp160 antigen without any added adjuvant will elicit an antibody response against gp160 (see table above). However, a much stronger antibody response is seen in groups of mice immunized with 1.0 μg gp160 adsorbed to alum adjuvant. A single dose of less than 0.1 μg of gp160 mixed with complete Freund's or formulated with alum will seroconvert ≥ 50% of the immunized mice. Although less so, the gp160 antigen was immunogenic in mice as an unformulated antigen at a pH 7.5 and a pH 6.0, but there was a loss of immunogenicity at the lower pH.

The ability of a candidate vaccine to elicit an immune response is a very important biological property. To confirm that the alum formulated gp160 vaccine was immunogenic in animals and to confirm that the alum adjuvant increased this immunogenicity, the following experiment was performed. On day 0, mice (groups of 10) were injected with a single dose (0.5 μg or 5.0 μg) of gp160 alone, gp160 adsorbed to alum or gp160 in complete Freund's adjuvant (CFA). On day 28 the mice were bled and the sera examined by ELISA (1:10 dilution) for the presence of antibodies to gp160.

Results from the sera drawn on day 28 are summarized in the table below. In all groups, greater than 50% of the mice showed seroconversion. At all the doses the number of seroconversions and the average serum absorbance readings ($OD_{450}$ nm at a 1:10 dilution in the ELISA assay) were higher with gp160 adsorbed to alum than those obtained in mice immunized with gp160 alone. These results demonstrate that the alum adjuvant significantly increased the immunogenicity of the gp160 antigen.

## 28 Days Post-Injection

| | 0.5 μg Dose | | 1.0 μg Dose | | 5.0 μg Dose | |
|---|---|---|---|---|---|---|
| | P/N[1] | Mean OD[2] | P/N | Mean OD | P/N | Mean OD |
| gp160 | 9/10 | .407 | 7/10 | .699 | 7/10 | .430 |
| gp160 (alum) | 9/10 | .547 | 8/10 | .797 | 10/10 | 1.347 |
| gp160 (CFA) | 10/10 | 1.130 | 10/10 | 1.967 | 10/10 | 1.317 |

[1] The number of mice that seroconverted (P) compared to total number tested (n) at 28 days after being immunized with 0.5 μg, 1 μg or 5 μg of VaxSyn[tm] HIV-1.

[2] The mean absorbance (OD 450) of the mice that seroconverted as measured by the sponsor's ELISA assay against gp160 at a 1:10 dilution of serum

## EXAMPLE 12

### Neutralization Data

HIV-1 neutralization assays are an accepted method to determine whether an antibody preparation will inhibit the HIV-1 virus from infecting susceptible human cultured lymphocyte cells. Antisera from animals immunized with gp160 were tested in an HIV-1 neutralization assay and the results summarized in the table below.

| Animal | Identification | Immunogen/Adjuvant | µg[1] | Neutralizing Titer[2] |
|--------|----------------|--------------------|-------|-----------------------|
| Rhesus | G55 | gp120/Alum | 16/8/8 | 1:80-1:160 |
| Rhesus | H55 | gp120/Alum | 16/8/8 | 1:80-1.160 |
| Rhesus | L55 | gp160/Alum | 16/8/8 | ≥ 1:80 |
| Mice | Pool 3 | gp120/Freunds | .25/.25.25 | 1:40-1.80 |
| Mice | Pool 8 | gp160/Freunds | .1/.1/.1 | 1:40-1.80 |
| G. Pig | purified IgG | gp/160/Freunds | 10/10/10 | 1:320 |

[1] Micrograms of gp160 or gp120 administered during the first/second/third immunization.

[2] The highest dilution of antisera that will inhibit the infection by 50% relative to HIV-1 infected cells that were exposed to serum from non-immunized animals

Guinea pigs, rabbits and rhesus monkeys have also been immunized with gp160 (using alum or Freunds as an adjuvant). In general, the immunization of these animals has produced a good antibody response against the HIV-1 envelope proteins.

## EXAMPLE 13

### Immunogenicity in Chimpanzees

Genetically, the chimpanzee is man's closest relative and is currently the only animal model for infection of HIV-1. In a safety/immunogenicity trial in three chimpanzees, two chimpanzees were immunized with 40 µg or 80 µg gp160 in alum formulated vaccine. Each received a booster immunization at 4 weeks with 40 ug and 80 µ g gp 160, respectively. A control animal was vaccinated at the same time with 1 ml saline solution. Weekly serum samples were analyzed from each of the three chimpanzees for antibodies to gp160 and to HIV-1 viral antigens using three immunological assays, an ELISA assay against purified gp160 developed by MicroGeneSys, Inc., Western Blot analysis, and a commercial HIV-1 ELISA assay. The results of these analyses are described below:

### ELISA (MGSearch HIV 160).

The ELISA assay, MGSearch HIV 160, MGSearch being a trademark of MicroGeneSys, Inc. of West Haven, Connecticut, U.S.A., is an immunoadsorbant assay against gp160 and is described in copending coassigned U.S. patent Application Serial No. 920,197. Serum samples taken before immunization and for the 11 weeks following the primary immunization were diluted from 1:10 to 1:100,000 and then incubated with nitrocellulose strips containing 100 ng purified gp160 in a spot. The end point dilution titer is the highest dilution in which the test was positive for anti-gp160 antibody as detected with a goat anti-human IgG-alkali phosphatase conjugate. The serum samples from the control animal and from the preimmune sera of the immunized animal were negative. The chimp which received and 80 µg dose was positive at a 1:100 dilution by week 2 and the chimp which received a 40 µg dose was positive at 1:10 dilution by week 4. The antibody titers to gp160 continued to increase until week 5, at which time the end point dilution titers were approximately 1:100,000 and 1:2,000, respectively. The antibody titer in both animals dropped just slightly during weeks 6-11. This type of response is similar both quantitatively and qualitatively to antibody responses commonly observed in chimps that have been vaccinated with a human Hepatitis B Virus Vaccine.

### Commercial ELISA Test

It was clear from the MGSearch HIV 160 ELISA and Western blot analyses of sera from the VaxSyn immunized chimpanzees, VaxSyn being a trademark of MicroGeneSys, Inc. for AIDS vaccine, that they had seroconverted and have antibodies against the recombinant gp160. To determine if they were also making anti-HIV antibody which recognized the native viral envelope proteins, the preimmune sera and sera from weeks 1 through 11 were tested in a licensed, commercial ELISA test kit LAV EIA test kit, LAV EIA being a trademark of Genetic System Corporation, Seattle, Washington, U.S.A. The animal immunized with 80 μg gp160 was positive at a 1:100 dilution by week 2 and continued to show an increase in antibody level through week 6. The animal immunized with 40 μg was positive at a 1:100 dilution by week 6.

## EXAMPLE 14

Distribution of Antibodies Between gp120 and gp41

It is important to determine whether the antibody responses against gp160 in a vaccinated animal is directed against gp41 or gp120 or both. A variety of immunological methods, including radioimmuno-precipitation (RIP), immunofluoresence (IF), Western blot analysis, and quantitative ELISA against three different recombinant envelope antigens were employed to detect and measure for the distribution of antibodies against various regions of the HIV-1 envelope proteins. Fig 6 summarizes the immunoreactivity of three different recombinant antigens (1) gp120-γ (truncated recombinant HIV-1 gp120 with about 40 amino acids missing from the C-terminus of the molecule), (2) gp120 (full length recombinant HIV-1 gp120; and (3) gp160. Human sera from 50 HIV-1 antibody positive individuals and 3 pooled human sera were highly reactive with gp160, moderately reactive with gp120 and little or no antibody reacted with truncated gp120. It is likely that the truncated gp120, which represents more than 90% of the HIV-1 external glycoprotein, contains protective determinants. The observation that human AIDS positive sera have few antibodies to this region of the envelope is consistent with the fact that the immune response to viral infection is not fully protective and that human positive sera usually exhibit a low-level of neutralizing activity in vitro. In contrast, rhesus monkeys immunized with either the gp160 immunogen or with the truncated gp120 have antibodies that react strongly with the truncated gp 120 portion of the HIV-1 envelope. This difference in distribution of antibody recognition sites along the viral envelope and the higher titers observed in the monkeys may account for the fact that the monkey sera had high neutralizing titers. A quantitative assessment of the immunoreactivity of these three recombinant envelope antigens with human and immune rhesus sera is presented in Fig. 7. All the monkey sera tested had high titer antibody against the truncated gp120 antigen (gp120-γ), including those from animals immunized with gp160.

The following is a listing of and a more complete identification of the reference cited hereinabove:

### References

Adachi, A. H.E. Gendelman, S. Koenig, T. Folks, R. Willey, A. Rabson, and M. Martin. 1986. Production of acquired immunodeficiency syndrome-associated retrovirus in human and nonhuman cells transfected with an infectious molecular clone J. Virol. 59:284-291.

Barin F, MF McLane, JS Allan, et al. Virus envelope protein of HTLV-III represents major targen antigen for antibodies in AIDS patient **Science.** 228:1094-1096.

Barré-Sinoussi F, JC Chermann, F Rey, et al. 1983. Isolation of T-Lymphotropic Retrovirus from a Patient at Risk for Acquired Immune Deficiency Syndrome. **Science.** 220:868-871.

Bomford R. 1985. Adjuvants. in **Animal Cell Biotech.** Vol. 2:235-250. Academic Press Inc. (London) Ltd.

Chakrabarti, S, M Robert-Guroff, F Wong-Stall, RC Gallo, B Moss. 1986. Expression of the HTLV-III envelope gene by a recombinant vaccinia virus. **Nature.** 320:535-537.

Cochran MA, BL Ericson, JD Knell, GE Smith. 1987. Use of baculovirus recombinants as a general method for the production of subunit vaccines. in **Vaccines 87.** Cold Springs Harbor Press, in press.

Fauci, Anthony S. 1986. Current issues in developing a strategy for dealing with the acquired immunodeficiency syndrome. Proc. Natl. Acad. Sci. USA. 83:9278-9238.

Feorino, PM, VS Kalyanaranman, HW Haverkos, et al. 1984. Lymphadenopathy associated virus infection of a blood donor-recipient par with acquired immunodeficiency syndrome. **Science.** 225:69-72.

Francis DP, JC Petricciani. 1985. The prospects for and pathways toward a vaccine for AIDS. **New Eng. Journal of Med.** 1586-1590.

Kennedy RC, RD Henkel, D Pauleti, JS Allan, TH Lee, M Essex, GR Dreesman. 1986. Antiserum peptide recognizes the HTLV-III envelope glycoprotein **Science.** 231:1556-1559.

Kieny MP, G Rautmann, D Schmitt, K Dott, S Wain-Hobson, M Alikzon, M Girard, S Chamaret, A Laurent, L Montagnier, and J-P Lecocq. 1986. AIDS virus *env* protein expressed from a recombinant vaccinia virus. **Biotechnology.** 4:790-795.

Hu, S-L, SG Kosowski, JM Dalrymple. 1986. Expression of AIDS virus envelope gene in recombinant vaccinia viruses. **Nature.** 320:537-540. -

Lasky, LA JE Groopman, CW Fennie, PM Benz, DJ Capon, DJ Dowabenko, GR Nakamura, WM Nunes, ME Renz PW Berman. 1986. **Science.** 233:209-212.

Levy, JA, AD Hoffman, SM Kramer, JA Landis, JM Shimabukurs, and LS Oshiro. 1984. Isolation of lymphcytopathic retroviruses from San Francisco patients with AIDS. **Science.** 225:840-842.

Popovic,M, M Sarangadharan, E Read, and R Gallo. 1984. Detection, Isolation and Continuous Production of Cytopathic Retroviruses (HTLV-III) from patients with AIDS and at risk for AIDS. **Science.** 224:497-500.

Robey WG, LO Arthur, TJ Matthews *et al.* 1986. Prospect for prevention of human immunodeficiency virus infection: purified 120-kDA envelope glycoprotein induces neutralizing antibody. **Proc. Natl. Acad. USA.** 83:7023-7027.

Salk, J. 1987. Prospects for the control of AIDS through post-exposure immunization. **Nature.** 327:473-476.

Putney, SD., Matthews, T.J., Robey, W.G., Lynn, D.L., Robert-Guroff, M., Muller, W.T., Langlois, A.J., Ghrayeb, J., Petteway, S.R., Jr., Weinhold, K.J., Fischinger, P.J., Wong-Staahl, F., Gallo, R.C., and Bolognesi, D.P. 1986. HTLV-III/LAV Neutralizing antibodies to an *E. coli*-produced fragment of the virus envelope. **Science.** 234:1392-1395.

Vogt, M. and M.S. Hirsch. 1986. Prospects for the prevention and therapy of infections with the human immunodeficiency virus. **Reviews of Infectious Disease.** 8:991-1000.

Wain-Hobson, S., Sonigo, P., Danos, O., Cole, S., and Alizon, M. Nucleotide Sequence of the AIDS virus, LAV. 1985. Cell 40:9-17.

## Claims

1. Undenatured or native HIV-1 envelope protein.

2. Recombinant undenatured or native HIV-1 envelope protein of Claim 1.

3. Substantially biologically pure HIV-1 envelope protein in accordance with Claim 1.

4. Substantially biologically pure HIV-1 envelope protein in accordance with Claim 1 of a purity and quality useful as a vaccine for humans.

5. Substantially biologically pure gp160 protein in accordance with Claim 1.

6. Recombinant substantially pure HIV-1 envelope protein in accordance with Claim 1 of a purity and quality useful as a vaccine for humans.

7. A substantially non-pyrogenic vaccine composition containing a protein in accordance with Claim 1 together with an adjuvant.

8. A composition in accordance with Claim 7 wherein said adjuvant is particle-form aluminum phosphate.

9. A composition in accordance with Claim 7 wherein said adjuvant is particle-form aluminum hydroxide.

10. A composition for the treatment of acquired immunodeficiency syndrome (AIDS) compris ing a minor amount of a protein in accordance with Claim 1 together with a carrier.

11. gp160 HIV-1 envelope protein in accordance with Claim 1.

12. Recombinant gp160 HIV-1 envelope protein in accordance with Claim 1.

13. A recombinant DNA molecule for expressing HIV-1 envelope protein in accordance with Claim 1.

14. A DNA molecule of Figs. 4a-4k herein for expressing HIV-1 envelope protein in accordance with Claim 1.

15. A recombinant DNA molecule containing the DNA sequence for expressing the HIV-1 envelope protein of Claim 1.

16. A recombinant DNA molecule in accordance with Claim 15 containing a DNA sequence for expressing gp160 HIV-1 envelope protein.

17. A DNA molecule in accordance with AC3046 herein.

18. Substantially biologically pure undenatured or native HIV-1 envelope protein in accordance with Claim 1 recovered after expression in a host insect cell.

19. Substantially biologically pure undenatured or native gp160 HIV-1 envelope protein in accordance with Claim 18.

20. Recombinant substantially biologically pure undenatured or native HIV-1 envelope protein expressed by Ac3046 herein in accordance with Claim 18.

21. The recombinant DNA molecule Ac3046 herein in accordance with Claim 17 for the expression of undenatured or native gp160 envelope protein of HIV-1 in an insect cell.

22. A method of treating a human patient suffering from AIDS which comprises administering to the patient a therapeutically effective amount of a protein in accordance with Claim 1.

23. A method for preventing AIDS in a human which comprises administering to said human an effective prophylactic amount of a protein in accordance with Claim 4.

24. A method of recovering and purifying recombinant HIV-1 envelope protein material expressed in a cell which comprises after expression of said protein material recovering and washing the cells, lysing the cells, subjecting the resulting lysate to a lectin affinity chromatography followed by gel filtration chromatography and dialysis and recovering the resulting purified HIV-1 envelope protein material.

25. A method in accordance with Claim 24 wherein said cells containing the to-be-recovered HIV-1 envelope protein material therein are subjected to washing in a buffer solution containing 50 nM Tris buffer at a pH 7.5 and 1nM EDTA and 1% Triton X-100.

26. A method in accordance with Claim 24 wherein cells lysis is carried out by sonication in a 50 nM Tris buffer at a pH in the range about 8.0-8.5 and 4% deoxycholate and 1% beta mercaptoethanol.

27. A method in accordance with Claim 24 wherein said gel filtration is carried out to a column containing Sephacryl resin.

28. A method in accordance with Claim 24 wherein said lectin affinity chromatography is carried out employing lentil lectin.

29. A method in accordance with Claim 24 wherein the resulting recovered purified HIV-1 envelope protein material is agglomerated or assembled into particles of about ≥ 2,000,000 molecular weight.

30. A method in accordance with Claim 24 wherein said HIV-1 envelope protein material is gp160.

31. A method in accordance with Claim 24 wherein said HIV-1 envelope protein material is gp160 which is agglomerated or assembled into particles having a molecular weight of about ≥ 2,000,000.

32. Undenatured or native HIV-1 envelope protein material in accordance with Claim 1 in finely divided particle-form and having a molecular weight of about ≥ 2,000,000.

33. Undenatured or native HIV-1 envelope protein material in accordance with Claim 32 wherein said protein material is gp160.

34. Undenatured or native HIV-1 envelope protein material in accordance with Claim 1 having a substantially spherical particle-form in the range about 30-100nM.

35. Undenatured or native HIV-1 envelope protein material in accordance with Claim 1 wherein said protein material is gp160.

36. An immunogen composition comprising undenatured or native HIV-1 envelope protein material complexed with alum wherein said protein material is present therein at a concentration of about 40μg/ml of said composition.

37. A method of complexing HIV-1 envelope protein material with alum wherein said protein is complexed with said alumn at a pH of about 7.0-7.2.

38. A method in accordance with Claim 37 wherein said HIV-1 envelope protein material is gp160.

## ISOLATION AND ENGINEERING OF HIV-1 env GENE

FIG. 1

synthetic oligo

```
GlyArgValLysGluLysTyrGlnHisLeuTrpArgTrp
CCCGGGCGTGTGAAGGAGAAGTACCAACACCTGTGGCGTTGG

GlyTrpLysTrpGlyThrMetLeuLeuGlyIleLeuMetIle
GGCTGGAAGTGGGGCACCATGCTGCTGGGCATCCTGATGATC

CysSerAlaThrGluLysLeuTrpValThrValTyrTyr
TGTAGCGCTACCGAGAAGCTGTGGGTGACCGTGTACTACG
```

CONSTRUCTION OF RECOMBINATION VECTOR p3046

FIG. 2

pMGS3

Xhol→
1.90

Sall

EcoRV   4.00
3.90

← EcoRI
6.70

Sall 5.65
BamHI
5.56

polyhedrin promoter: ATG CCC GGG GGT ACC AGA TCT TAA TTA ATT AAGT

Smal   Kpnl   BglII

p1774

← Kpnl
← BamHI

env

Smal   Kpnl

GlyArgValLysGluLysTyrGlnHisLeuTrpArgTrp
CCCGGGCGTGTGAAGGAGAAGTACCAACACCTGTGGCGTTGG

GlyTrpLysTrpGlyThrMetLeuLeuGlyIleLeuMetIle
GGCTGGAAGTGGGGCACCATGCTGCTGGGCATCCTGATGATC

CysSerAlaThrGluLysLeuTrpValThrValTyrTyr
TGTAGCGCTACCGAGAAGCTGTGGGTGACCGTGTACTACG

+ Sma/Bgl          + Sma/Bam

p3046

← BamHI/Bgl

env

Smal   Kpnl

FIG. 3

NUCLEOTIDE SEQUENCE OF DNA FLANKING THE Ac3046 gpl60 CODING SEQUENCES


TGCTGATATC ATGGAGATAA TTAAAATGAT AACCATCTCG CAAATAAATA
-100

AGTATTTTAC TGTTTTCGTA ACAGTTTTGT AATAAAAAAA CCTATAAATA
-50

<u>ATG</u> -----/3046/----->TAATTAATTAA GT ACC GAC TCT GGT GAA GAG
+1                            +2253

GAG GAA ATT CTC CTT GAA GTT TCC CTG GTG TTC AAA GTA AAG GAG
+2287

TTT GCA CCA GAC GCA CCT CTG TTC ACT GGT CCG GCG TAT TAA
+2332                                                    +2374

FIG. 4a

NUCLEOTIDE SEQUENCE AND PREDICTED AMINO ACID SEQUENCE OF
3046 OPEN READING FRAME

With enzymes:

```
              SS
      AHNNccSS          R                          B    B N
      vpccrrem          s                          b    a l
      aaiiFFca          a                          v    n a
      12111111          1                          1    1 4
        //////
      ATGCCCGGGCGTGTGAAGGAGAAGTACCAACACCTGTGGCGTTGGGGCTGGAAGTGGGGC
  1   ---------+---------+---------+---------+---------+---------+ 60
      TACGGGCCCGCACACTTCCTCTTCATGGTTGTGGACACCGCAACCCCGACCTTCACCCCG
a:    MetProGlyArgValLysGluLysTyrGlnHisLeuTrpArgTrpGlyTrpLysTrpGly -


      B      F B              SS    F                        B
      sF     Nn s             af    nP  HH   B    A           sM
      po     lu t             ua    us  ha   b    l           ta
      1k     a4 X             3N    4t  ae   v    u           Ee
      21     3H 1             A1    H1  12   1    1           23
       /                       /                              /
      ACCATGCTGCTGGGCATCCTGATGATCTGCAGCGCTACCGAGAAGCTGTGGGTGACCGTG
 61   ---------+---------+---------+---------+---------+---------+ 120
      TGGTACGACGACCCGTAGGACTACTAGACGTCGCGATGGCTCTTCGACACCCACTGGCAC
a:    ThrMetLeuLeuGlyIleLeuMetIleCysSerAlaThrGluLysLeuTrpValThrVal -


                                               S              S
       R H    B NRK                            f              f
       s p    a lsp                            a              a
       a h    n aan                            N              N
       1 1    1 411                            1              1
         /
      TACTACGGGGTACCTGTGTGGAAGGAAGCAACCACCACTCTATTTTGTGCATCAGATGCT
121   ---------+---------+---------+---------+---------+---------+ 180
      ATGATGCCCCATGGACACACCTTCCTTCGTTGGTGGTGAGATAAAACACGTAGTCTACGA
a:    TyrTyrGlyValProValTrpLysGluAlaThrThrThrLeuPheCysAlaSerAspAla -
```

FIG. 4b

```
                  T                S                N
        N M       t   R            a  H            Ns      R
        d n       h   s            u  a            lp      s
        e l       3   a            9  e            aH      a
        1 1       2   1            6  3            31      1

        AAAGCATATGATACAGAGGTACATAATGTTTGGGCCACACATGCCTGTGTACCCACAGAC
    181 ---------+---------+---------+---------+---------+---------+ 240
        TTTCGTATACTATGTCTCCATGTATTACAAACCCGGTGTGTACGGACACATGGGTGTCTG
 a:     LysAlaTyrAspThrGluValHisAsnValTrpAlaThrHisAlaCysValProThrAsp -


                                                     N
                              M                 A    Ns
                              a                 f    lp
                              e                 l    aH
                              3                 3    31
                                                        /
        CCCAACCCACAAGAAGTAGTATTGGTAAATGTGACAGAAAATTTTAACATGTGGAAAAAT
    241 ---------+---------+---------+---------+---------+---------+ 300
        GGGTTGGGTGTTCTTCATCATAACCATTTACACTGTCTTTTAAAATTGTACACCTTTTTA
 a:     ProAsnProGlnGluValValLeuValAsnValThrGluAsnPheAsnMetTrpLysAsn -


        S                                             S
        fN            M       N N                     a       B
        al            n       s l                     u       i
        Na            l       i a                     3       n
        13            1       1 3                     A       1

        GACATGGTAGAACAGATGCATGAGGATATAATCAGTTTATGGGATCAAAGCCTAAAGCCA
    301 ---------+---------+---------+---------+---------+---------+ 360
        CTGTACCATCTTGTCTACGTACTCCTATATTAGTCAAATACCCTAGTTTCGGATTTCGGT
 a:     AspMetValGluGlnMetHisGluAspIleIleSerLeuTrpAspGlnSerLeuLysPro -


                                          A   BH
        N                       D         D   p    sg              M
        1                       r         r   a    pi              b
        a                       a         a   L    1A              o
        3                       3         1   1    21              2
                                                       /
        TGTGTAAAATTAACCCCACTCTGTGTTAGTTTAAAGTGCACTGATTTGAAGAATGATACT
    361 ---------+---------+---------+---------+---------+---------+ 420
        ACACATTTTAATTGGGGTGAGACACAATCAAATTTCACGTGACTAAACTTCTTACTATGA
 a:     CysValLysLeuThrProLeuCysValSerLeuLysCysThrAspLeuLysAsnAspThr -
```

FIG. 4c

```
           AATACCAATAGTAGTAGCGGGAGAATGATAATGGAGAAAGGAGAGATAAAAAACTGCTCT
       421 ---------+---------+---------+---------+---------+---------+ 480
           TTATGGTTATCATCATCGCCCTCTTACTATTACCTCTTTCCTCTCTATTTTTTGACGAGA
        a: AsnThrAsnSerSerSerGlyArgMetIleMetGluLysGlyGluIleLysAsnCysSer -


                           T
                           t                B N
                           h                s s
                           3                m i
                           2                1 1
           TTCAATATCAGCACAAGCATAAGAGATAAGGTGCAGAAAGAATATGCATTCTTTTATAAA
       481 ---------+---------+---------+---------+---------+---------+ 540
           AAGTTATAGTCGTGTTCGTATTCTCTATTCCACGTCTTTCTTATACGTAAGAAAATATTT
        a: PheAsnIleSerThrSerIleArgAspLysValGlnLysGluTyrAlaPhePheTyrLys -


                    R                   A                M        D
                    s                   l                a        d
                    a                   u                e        e
                    1                   1                3        1
           CTTGATATAGTACCAATAGATAATACCAGCTATAGGTTGATAAGTTGTAACACCTCAGTC
       541 ---------+---------+---------+---------+---------+---------+ 600
           GAACTATATCATGGTTATCTATTATGGTCGATATCCAACTATTCAACATTGTGGAGTCAG
        a: LeuAspIleValProIleAspAsnThrSerTyrArgLeuIleSerCysAsnThrSerVal -


                                                                  B
                M      HHS                                        SAsHN
                n      aat                                        evppc
                l      eeu                                        calai
                1      131                                        11221
                       //                                         ///
           ATTACACAGGCCTGTCCAAAGGTATCCTTTGAGCCAATTCCCATACATTATTGTGCCCCG
       601 ---------+---------+---------+---------+---------+---------+ 660
           TAATGTGTCCGGACAGGTTTCCATAGGAAACTCGGTTAAGGGTATGTAATAACACGGGGC
        a: IleThrGlnAlaCysProLysValSerPheGluProIleProIleHisTyrCysAlaPro -


                SS         H                            S
                NccSS      i                            Aa      NR
                crrem      n              M             vu      ls
                iFFca      f              a             a9      aa
                11111      1              e             26      31
                /////                    2              /
           GGTGGTTTTGCGATTCTAAAATGTAATAATAAGACGTTCAATGGAACAGGACCATGTACA
       661 ---------+---------+---------+---------+---------+---------+ 720
           CCACCAAAACGCTAAGATTTTACATTATTATTCTGCAAGTTACCTTGTCCTGGTACATGT
        a: GlyGlyPheAlaIleLeuLysCysAsnAsnLysThrPheAsnGlyThrGlyProCysThr -
```

FIG. 4d

```
                  H
    R      R      Ni     'HH.
    s      s      ln      aa
    a      a      af      ee
    1      1      31      13
                          /
    AATGTCAGCACAGTACAATGTACACATGGAATCAGGCCAGTAGTATCAACTCAACTGCTG
721 ---------+---------+---------+---------+---------+---------+ 780
    TTACAGTCGTGTCATGTTACATGTGTACCTTAGTCCGGTCATCATAGTTGAGTTGACGAC
a:  AsnValSerThrValGlnCysThrHisGlyIleArgProValValSerThrGlnLeuLeu -


                                      S
           M                  M  M  BaX
           a        .         b  b  guh
           e                  o  o  13o
           1                  2  2  2A2
                                   //
    TTAAATGGCAGTCTAGCAGAAGAAGATGTAGTAATTAGATCTGCCAATTTCACAGACAAT
781 ---------+---------+---------+---------+---------+---------+ 840
    AATTTACCGTCAGATCGTCTTCTTCTACATCATTAATCTAGACGGTTAAAGTGTCTGTTA
a:  LeuAsnGlySerLeuAlaGluGluAspValValIleArgSerAlaAsnPheThrAspAsn -


                  N
    R      AsP                        R
    s      lpv                        s
    a      uBu                        a
    1      122                        1
           //
    GCTAAAACCATAATAGTACAGCTGAACACATCTGTAGAAATTAATTGTACAAGACCCAAC
841 ---------+---------+---------+---------+---------+---------+ 900
    CGATTTTGGTATTATCATGTCGACTTGTGTAGACATCTTTAATTAACATGTTCTGGGTTG
a:  AlaLysThrIleIleValGlnLeuAsnThrSerValGluIleAsnCysThrArgProAsn -


                          SBS
           M              AFNascS              M
           n              vilutre              a
           1              ana9NFc              e
           1              2146111              3
                          /// //
    AACAATACAAGAAAAAGTATCCGTATCCAGAGGGGACCAGGGAGAGCATTTGTTACAATA
901 ---------+---------+---------+---------+---------+---------+ 960
    TTGTTATGTTCTTTTTCATAGGCATAGGTCTCCCCTGGTCCCTCTCGTAAACAATGTTAT
a:  AsnAsnThrArgLysSerIleArgIleGlnArgGlyProGlyArgAlaPheValThrIle -
```

FIG. 4e

```
                              T
                         M    t                    B
                         a    h                     s
                         e    3                     m
                         3    2                     1
     GGAAAAAATAGGAAATATGAGACAAGCACATTGTAACATTAGTAGAGCAAAATGGAATGCC
961  ---------+---------+---------+---------+---------+---------+ 1020
     CCTTTTTATCCTTTATACTCTGTTCGTGTAACATTGTAATCATCTCGTTTTACCTTACGG
a:   GlyLysIleGlyAsnMetArgGlnAlaHisCysAsnIleSerArgAlaLysTrpAsnAla -

         D          AMN
         r          lah
         a          uee
         l          111
                     /
     ACTTTAAAACAGATAGCTAGCAAATTAAGAGAACAATTTGGAAATAATAAAACAATAATC
1021 ---------+---------+---------+---------+---------+---------+ 1080
     TGAAATTTTGTCTATCGATCGTTTAATTCTCTTGTTAAACCTTTATTATTTTGTTATTAG
a:   ThrLeuLysGlnIleAlaSerLysLeuArgGluGlnPheGlyAsnAsnLysThrIleIle -

                         PS
     M   DM          ADFMNNpa    M              M
     n   ds          vrinlluu    a              n
     l   et          aanlaaM9    e              l
     1   12          22114416    3              1
         /           ///////
     TTTAAGCAATCCTCAGGAGGGGACCCAGAAATTGTAACGCACAGTTTTAATTGTGGAGGG
1081 ---------+---------+---------+---------+---------+---------+ 1140
     AAATTCGTTAGGAGTCCTCCCCTGGGTCTTTAACATTGCGTGTCAAAATTAACACCTCCC
a:   PheLysGlnSerSerGlyGlyAspProGluIleValThrHisSerPheAsnCysGlyGly -

                         RS             RS
                         sc             sc
                         aa             aa
                         11             11
                          /              /
     GAATTTTTCTACTGTAATTCAACACAACTGTTTAATAGTACTTGGTTTAATAGTACTTGG
1141 ---------+---------+---------+---------+-----;---+---------+ 1200
     CTTAAAAAGATGACATTAAGTTGTGTTGACAAATTATCATGAACCAAATTATCATGAACC
a:   GluPhePheTyrCysAsnSerThrGlnLeuPheAsnSerThrTrpPheAsnSerThrTrp -
```

FIG. 4f

```
                              E                    E
             RS               c        M           c        N
             sc               o        a           o        l
             aa               5        e           5        a
             11               7        3           7        3
              /
          AGTACTGAAGGGTCAAATAACACTGAAGGAAGTGACACAATCACACTCCCATGCAGAATA
    1201  ---------+---------+---------+---------+---------+---------+ 1260
          TCATGACTTCCCAGTTTATTGTGACTTCCTTCACTGTGTTAGTGTGAGGGTACGTCTTAT
 a:       SerThrGluGlySerAsnAsnThrGluGlySerAspThrIleThrLeuProCysArgIle -


                        N
             A         Ns
             f         lp                                           M
             l         aH                                           n
             3         31                                           1
              /                                                     1
          AAACAATTTATAAACATGTGGCAGGAAGTAGGAAAAGCAATGTATGCCCCTCCCATCAGT
    1261  ---------+---------+---------+---------+---------+---------+ 1320
          TTTGTTAAATATTTGTACACCGTCCTTCATCCTTTTCGTTACATACGGGGAGGGTAGTCA
 a:       LysGlnPheIleAsnMetTrpGlnGluValGlyLysAlaMetTyrAlaProProIleSer -


                                             F
             B         S                     n
             b         s                     u
             v         p                     4
             1         1                     H
          GGACAAATTAGATGTTCATCAAATATTACTGGGCTGCTATTAACAAGAGATGGTGGTAAT
    1321  ---------+---------+---------+---------+---------+---------+ 1380
          CCTGTTTAATCTACAAGTAGTTTATAATGACCCGACGATAATTGTTCTCTACCACCATTA
 a:       GlyGlnIleArgCysSerSerAsnIleThrGlyLeuLeuLeuThrArgAspGlyGlyAsn -


             E         S    S              BS
             c       AMNa  BaX    M   M   scG       M          F
             o       vblu  guh    n   n   trs       n          i
             5       aoa9  13o    1   1   NFu       1          n
             7       2246  2A2    1   1   111       1          1
                     //    //                /
          AACAACAATGGGTCCGAGATCTTCAGACCTGGAGGAGGCGATATGAGGGACAATTGGAGA
    1381  ---------+---------+---------+---------+---------+---------+ 1440
          TTGTTGTTACCCAGGCTCTAGAAGTCTGGACCTCCTCCGCTATACTCCCTGTTAACCTCT
 a:       AsnAsnAsnGlySerGluIlePheArgProGlyGlyGlyAspMetArgAspAsnTrpArg -
```

FIG. 4g

```
                                                                    SS
                                                                    et
                                                                    cy
                                                                    11
                                                                    /
      AGTGAATTATATAAATATAAAGTAGTAAAAATTGAACCATTAGGAGTAGCACCCACCAAG
1441  ---------+---------+---------+---------+---------+---------+ 1500
      TCACTTAATATATTTATATTTCATCATTTTTAACTTGGTAATCCTCATCGTGGGTGGTTC
  a:  SerGluLeuTyrLysTyrLysValValLysIleGluProLeuGlyValAlaProThrLys -


                      M                                   A         SS
                      b                                   l         et
                      o   .                               u         cy
                      2                                   1         11
                                                                    /
      GCAAAGAGAAGAGTGGTGCAGAGAGAAAAAAGAGCAGTGGGAATAGGAGCTTTGTTCCTT
1501  ---------+---------+---------+---------+---------+---------+ 1560
      CGTTTCTCTTCTCACCACGTCTCTCTTTTTTCTCGTCACCCTTATCCTCGAAACAAGGAA
  a:  AlaLysArgArgValValGlnArgGluLysArgAlaValGlyIleGlyAlaLeuPheLeu -


              F                 F       .
              n       BH        Hn              B         H R
              u       bg        hu         .. b          g s
              4       va        a4          v    .       a a
              H       11        1H             1          1 1
      GGGTTCTTGGGAGCAGCAGGAAGCACTATGGGCGCAGCGTCAATGACGCTGACGGTACAG
1561  ---------+---------+---------+---------+---------+---------+ 1620
      CCCAAGAACCCTCGTCGTCCTTCGTGATACCCGCGTCGCAGTTACTGCGACTGCCATGTC
  a:  GlyPheLeuGlyAlaAlaGlySerThrMetGlyAlaAlaSerMetThrLeuThrValGln -


                          F  F
              HH          n  n      BM ·B    D
              aa          u  u      bn  b    d
              ee          4  4      vl  v    e
              13          H  H      11  1    1
              /
      GCCAGACAATTATTGTCTGATATAGTGCAGCAGCAGAACAATTTGCTGAGGGCTATTGAG
1621  ---------+---------+---------+---------+------·--+---------+ 1680
      CGGTCTGTTAATAACAGACTATATCACGTCGTCGTCTTGTTAAACGACTCCCGATAACTC
  a:  AlaArgGlnLeuLeuSerAspIleValGlnGlnGlnAsnAsnLeuLeuArgAlaIleGlu -
```

EP 0 327 180 A2

FIG. 4h

```
                  S                          S    BS   TH   BS
                  f                          f    Gsc  ti   sc
                  a                          a    str  hn   tr
                  N                          N    uNF  3f   NF
                  1                          1    111  21   11
                                                      /         /
        CAACAGCATCTGTTGCAATCTACAGTCTGGGGCATCAAACAACTCCAGGCAAGAATCCTG
  1681  ---------+---------+---------+---------+---------+---------+ 1740
        GTTGTCGTAGACAACGTTAGATGTCAGACCCCGTAGTTTGTTGAGGTCCGTTCTTAGGAC
a:      GlnGlnHisLeuLeuGlnSerThrValTrpGlyIleLysGlnLeuGlnAlaArgIleL.. -


                  S                   BS
                  a            BA    Ssc
                  u            il    etr
                  3            nu    cNF
                  A            11    111
                                      /
        GCTGTGGAAAGATACCTAAAGGATCAACAGCTCCTGGGGATTTGGGGTTGCTCTGGAAAA
  1741  ---------+---------+---------+---------+---------+---------+ 1800
        CGACACCTTTCTATGGATTTCCTAGTTGTCGAGGACCCCTAAACCCCAACGAGACCTTTT
a:      AlaValGluArgTyrLeuLysAspGlnGlnLeuLeuGlyIleTrpGlyCysSerGlyLys -


                  M       SS          MB
                  m       et          as
                  e       cy          em
                  1       11          11
                          /
        CTCATTTGCACCACTGCTGTGCCTTGGAATGCTAGTTGC.GTAATAAATCTCTGGAACAG
  1801  ---------+---------+---------+---------+---------+---------+ 1860
        GAGTAAACGTGGTGACGACACGGAACCTTACGATCAACCTCATTATTTAGAGACCTTGTC
a:      LeuIleCysThrThrAlaValProTrpAsnAlaSerTrpSerAsnLysSerLeuGluGln -


                    BS                                        H
                  N sc          F    F                        i A
                  1 tr          i    o                        n l
                  a NF          n    k                        d u
                  3 11          1    1                        3 1
                    /
        ATTTGGAATAACATGACCTGGATGGAGTGGGACAGAGAAATTAACAATTACACAAGCTTA
  1861  ---------+---------+---------+---------+---------+---------+ 1920
        TAAACCTTATTGTACTGGACCTACCTCACCCTGTCTCTTTAATTGTTAATGTGTTCGAAT
a:      IleTrpAsnAsnMetThrTrpMetGluTrpAspArgGluIleAsnAsnTyrThrSerLeu -
```

FIG. 4i

```
                         H
                         i              M
                         n              b
                         f              o
                         1              2
           ATACACTCCTTAATTGAAGAATCGCAAAACCAGCAAGAAAAGAATGAACAAGAATTATTG
      1921 ---------+---------+---------+---------+---------+---------+ 1980
           TATGTGAGGAATTAACTTCTTAGCGTTTTGGTCGTTCTTTTCTTACTTGTTCTTAATAAC
    a:     IleHisSerLeuIleGluGluSerGlnAsnGlnGlnGluLysAsnGluGlnGluLeuLeu -


           GAATTAGATAAATGGGCAAGTTTGTGGAATTGGTTTAACATAACAAATTGGCTGTGGTAT
      1981 ---------+---------+---------+---------+---------+---------+ 2040
           CTTAATCTATTTACCCGTTCAAACACCTTAACCAAATTGTATTGTTTAACCGACACCATA
    a:     GluLeuAspLysTrpAlaSerLeuTrpAsnTrpPheAsnIleThrAsnTrpLeuTrpTyr -


                         M                                           R
                         n                                           s
                         l                                           a
                         1                                           1
           ATAAAATTATTCATAATGATAGTAGGAGGCTTGGTAGGTTTAAGAATAGTTTTTGCTGTA
      2041 ---------+---------+---------+---------+---------+---------+ 2100
           TATTTTAATAAGTATTACTATCATCCTCCGAACCATCCAAATTCTTATCAAAAACGACAT
    a:     IleLysLeuPheIleMetIleValGlyGlyLeuValGlyLeuArgIleValPheAlaVal -


                         H
                         p
                         h
                         1
           CTTTCTATAGTGAATAGAGTTAGGCAGGGATATTCACCATTATCGTTTCAGACCCACCTC
      2101 ---------+---------+---------+---------+---------+---------+ 2160
           GAAAGATATCACTTATCTCAATCCGTCCCTATAAGTGGTAATAGCAAAGTCTGGGTGGAG
    a:     LeuSerIleValAsnArgValArgGlnGlyTyrSerProLeuSerPheGlnThrHisLeu -


                           PS    S
           M     AMS     ADFNNpa   aH                            M    M
           n     vne     vrilluu   ua                            b    b
           l     alc     aanaaM9   9e                            o    o
           1     111     2214416   63                            2    2
                   /      //////
           CCAATCCCGAGGGGACCCGACAGGCCCGAAGGAATAGAAGAAGAAGGTGGAGAGAGAGAC
      2161 ---------+---------+---------+---------+---------+---------+ 2220
           GGTTAGGGCTCCCCTGGGCTGTCCGGGCTTCCTTATCTTCTTCTTCCACCTCTCTCTCTG
    a:     ProIleProArgGlyProAspArgProGluGlyIleGluGluGluGlyGlyGluArgAsp -
```

FIG. 4j

```
           S                     S
     B    aX     T         aX    B
     i    uh     a         uh    i
     n    3o     q         3o    n
     1    A2     1         A2    1
            /                 /
     AGAGACAGATCCATTCGATTAGTGAACGGATCTTAATTAATTAA
2221 ---------+---------+---------+---------+---- 2264
     TCTCTGTCTAGGTAAGCTAATCACTTGCCTAGAATTAATTAATT
a:   ArgAspArgSerIleArgLeuValAsnGlySerEndLeuIleLys-
```

Enzymes that do cut:

| | | | | | | | | | | |
|---|---|---|---|---|---|---|---|---|---|---|
| Afl3 | Alu1 | ApaL1 | Ava1 | Ava2 | Ban1 | Bbv1 | Bgl2 | Bin1 | Bsm1 | Bsp12 |
| BstE2 | BstN1 | BstX1 | Dde1 | Dra1 | Dra2 | Dra3 | Eco57 | Fin1 | Fnu4H | Fok1 |
| Gsu1 | Hae1 | Hae2 | Hae3 | Hga1 | HgiA1 | Hha1 | Hind3 | Hinf1 | Hpa2 | Hph1 |
| Kpn1 | Mae1 | Mae2 | Mae3 | Mbo2 | Mme1 | Mnl1 | Mst2 | Nci1 | Nde1 | Nhe1 |
| Nla3 | Nla4 | Nsi1 | NspB2 | NspH1 | PpuM1 | Pst1 | Pvu2 | Rsa1 | Sau3A | Sau96 |
| Sca1 | ScrF1 | Sec1 | SfaN1 | Sma1 | Ssp1 | Stu1 | Sty1 | Taq1 | Tth32 | Xho2 |

Enzymes that do not cut:

| | | | | | | | | | | |
|---|---|---|---|---|---|---|---|---|---|---|
| Aat2 | Acc1 | Afl2 | Aha2 | Apa1 | Asu2 | Avr2 | Ball | BamH1 | Ban2 | Bbv2 |
| Bcl1 | Bgl1 | BspH1 | BspM1 | BspM2 | BssH2 | Cfr1 | Cfr10 | Cla1 | Dsa1 | Eco31 |
| EcoB | EcoK | EcoR1 | EcoRV | Esp1 | Fsp1 | Gdi2 | HgiE2 | Hinc2 | Hpa1 | Mlu1 |
| Nae1 | Nar1 | Nco1 | Not1 | Nru1 | PflM1 | PmaC1 | Pvu1 | Rsr2 | Sac1 | Sac2 |
| Sal1 | Sfi1 | SnaB1 | Spe1 | Sph1 | Spl1 | Tha1 | Tth31 | Xba1 | Xho1 | Xma3 |
| Xmn1 | | | | | | | | | | |

FIG. 4k

NUMBER OF OPEN READING FRAME BASES: 2253

NUMBER OF AMINO ACID CODONS: = 2253 + 3 = 751

| Amino Acid | Number | Weight. | Totals |
|---|---|---|---|
| GLY - | 53 | 75.1 | 3980.3 |
| GLU - | 41 | 147.1 | 6031.1 |
| ASP - | 25 | 133.1 | 3327.5 |
| VAL - | 48 | 117.1 | 5620.8 |
| ALA - | 37 | 89.1 | 3296.7 |
| ARG - | 39 | 174.1 | 6793.8 |
| SER - | 28 | 105.1 | 2942.8 |
| LYS - | 42 | 146.2 | 6140.4 |
| ASN - | 58 | 132.1 | 7661.8 |
| MET - | 17 | 149.2 | 2536.4 |
| ILE - | 57 | 131.2 | 7478.4 |
| THR - | 53 | 119.1 | 6312.3 |
| TRP - | 26 | 204.2 | 5309.2 |
| CYS - | 21 | 121.2 | 2545.2 |
| TYR - | 16 | 181.2 | 2899.2 |
| LEU - | 61 | 131.2 | 8003.2 |
| PHE - | 25 | 165.2 | 4130.0 |
| SER - | 26 | 105.1 | 2732.6 |
| GLN - | 38 | 146.2 | 5555.6 |
| HIS - | 11 | 155.2 | 1707.2 |
| PRO - | 29 | 115.1 | 3337.9 |

TOTALS: 751     98,342.4

$- H_2O$ (751 x 18)

Total estimated weight of
non-glycosylated polypeptide    = 84,824.4

Total Number of glycosylation sites: 28

x 2100 (wt per oligo saccharide)

Total Estimated Mol. Wt. of gp160    = 84,824.4 + 58800

= 143,624.

FIG. 5a

## COMPARISON OF LAV-1 AND RECOMBINANT Ac3046 gp160* SEQUENCE

The sequence and corresponding codons on the top lines are those predicted from the engineering and by Wain-Hobson et al. (1985). The sequence along the bottom of each line is that which was determined for Ac3046 from recombinant viral DNA.

FIG. 5b

```
170
Ile Arg Gly Lys Val Gln Lys Glu Tyr Ala Phe Phe Tyr Lys Leu Asp Ile Ile Pro Ile
ATA AGA GGT AAG GTG CAG AAA GAA TAT GCA TTT TTT TAT AAA CTT GAT ATA ATA CCA ATA    6790
||| ||| |A| ||| ||| ||| ||| ||| ||| ||| ||C ||| ||| ||| ||| ||| ||| G|| ||| |||
        Asp                             Phe                     Val
    190 Y
Asp Asn Asp Thr Thr Ser Tyr Thr Leu Thr Ser Cys Asn Thr Ser Val Ile Thr Gln Ala
GAT AAT GAT ACT ACC AGC TAT ACG TTG ACA AGT TGT AAC ACC TCA GTC ATT ACA CAG GCC    6850 .
||| ||| (-------)||| ||| ||| |G| ||| |T| ||| ||| ||| ||| ||| ||| ||| ||| ||| |||
              Arg         Ile
    210
Cys Pro Lys Val Ser Phe Glu Pro Ile Pro Ile His Tyr Cys Ala Pro Ala Gly Phe Ala
TGT CCA AAG GTA TCC TTT GAG CCA ATT CCC ATA CAT TAT TGT GCC CCG GCT GGT TTT GCG    6910
||| ||| ||| ||| ||| ||| ||| ||| ||| ||| ||| ||| ||| ||| ||| ||| |G| ||| ||| |||
                                                                  Gly
    230       Y               Y                               Y
Ile Leu Lys Cys Asn Asn Lys Thr Phe Asn Gly Thr Gly Pro Cys Thr Asn Val Ser Thr
ATT CTA AAA TGT AAT AAT AAG ACG TTC AAT GGA ACA GGA CCA TGT ACA AAT GTC AGC ACA    6970
||| ||| ||| ||| ||| ||| ||| ||| ||| ||| ||| ||| ||| ||| ||| ||| ||| ||| ||| |||
    250                                                   Y
Val Gln Cys Thr His Gly Ile Arg Pro Val Val Ser Thr Gln Leu Leu Leu Asn Gly Ser
GTA CAA TGT ACA CAT GGA ATT AGG CCA GTA GTA TCA ACT CAA CTG CTG TTG AAT GGC AGT    7030
||| ||| ||| ||| ||| ||| ||C ||| ||| ||| ||| ||| ||| ||| ||| ||| ||A ||| ||| |||
                          Ile                                 Leu
    270                           Y
Leu Ala Glu Glu Glu Val Val Ile Arg Ser Ala Asn Phe Thr Asp Asn Ala Lys Thr Ile
CTA GCA GAA GAA GAG GTA GTA ATT AGA TCT GCC AAT TTC ACA GAC AAT GCT AAA ACC ATA    7090
||| ||| ||| ||| ||T ||| ||| ||| ||| ||| ||| ||| ||| ||| ||| ||| ||| ||| ||| |||
              Asp
    290       Y                           Y                   Y
Ile Val Gln Leu Asn Gln Ser Val Glu Ile Asn Cys Thr Arg Pro Asn Asn Asn Thr Arg
ATA GTA CAG CTG AAC CAA TCT GTA GAA ATT AAT TGT ACA AGA CCC AAC AAC AAT ACA AGA    7150
||| ||| ||| ||| ||| AC| ||| ||| ||| ||| ||| ||| ||| ||| ||| ||| ||| ||| ||| |||
              Thr
    310
Lys Ser Ile Arg Ile Gln Arg Gly Pro Gly Arg Ala Phe Val Thr Ile Gly Lys Ile Gly
AAA AGT ATC CGT ATC CAG AGG GGA CCA GGG AGA GCA TTT GTT ACA ATA GGA AAA ATA GGA    7210
||| ||| ||| ||| ||| ||| ||| ||| ||| ||| ||| ||| ||| ||| ||| ||| ||| ||| ||| |||
    330               Y                           Y
Asn Met Arg Gln Ala His Cys Asn Ile Ser Arg Ala Lys Trp Asn Ala Thr Leu Lys Gln
AAT ATG AGA CAA GCA CAT TGT AAC ATT AGT AGA GCA AAA TGG AAT GCC ACT TTA AAA CAG    7270
||| ||| ||| ||| ||| ||| ||| ||| ||| ||| ||| ||| ||| ||| ||| ||| ||| ||| ||| |||
    350                           Y
Ile Ala Ser Lys Leu Arg Glu Gln Phe Gly Asn Asn Lys Thr Ile Ile Phe Lys Gln Ser
ATA GCT AGC AAA TTA AGA GAA CAA TTT GGA AAT AAT AAA ACA ATA ATC TTT AAG CAA TCC    7330
||| ||| ||| ||| ||| ||| ||| ||| ||| ||| ||| ||| ||| ||| ||| ||| ||| ||| ||| |||
    370
Ser Gly Gly Asp Pro Glu Ile Val Thr His Ser Phe Asn Cys Gly Gly Glu Phe Phe Tyr
TCA GGA GGG GAC CCA GAA ATT GTA ACG CAC AGT TTT AAT TGT GGA GGG GAA TTT TTC TAC    7390
||| ||| ||| ||| ||| ||| ||| ||| ||| ||| ||| ||| ||| ||| ||| ||| ||| ||| ||| |||
```

FIG. 5c

```
390 Y                           Y                    Y
Cys Asn Ser Thr Gln Leu Phe Asn Ser Thr Trp Phe Asn Ser Thr Trp Ser Thr Glu Gly
TGT AAT TCA ACA CAA CTG TTT AAT AGT ACT TGG TTT AAT AGT ACT TGG AGT ACT GAA GGG   7450
III III III III III III III III III III III III III III III III III III III III

410 Y
Ser Asn Asn Thr Glu Gly Ser Asp Thr Ile Thr Leu Pro Cys Arg Ile Lys Gln Phe Ile
TCA AAT AAC ACT GAA GGA AGT GAC ACA ATC ACA CTC CCA TGC AGA ATA AAA CAA TTT ATA   7510
III III III III III III III III III III III III III III III III III III III III

430
Asn Met Trp Gln Glu Val Gly Lys Ala Met Tyr Ala Pro Pro Ile Ser Gly Gln Ile Arg
AAC ATG TGG CAG GAA GTA GGA AAA GCA ATG·TAT GCC CCT CCC ATC AGC GGA CAA ATT AGA   7570
III III III III III III III III III III III III III III III IIT III III III III
                                                                Ser

450          Y                                                              Y
Cys Ser Ser Asn Ile Thr Gly Leu Leu Leu Thr Arg Asp Gly Gly Asn Asn Asn Asn Gly
TGT TCA TCA AAT ATT ACA GGG CTG CTA TTA ACA AGA GAT GGT GGT AAT AAC AAC AAT GGG   7630
III III III III III IIT III III III III III III III III III III III III III III
                     Thr

470
Ser Glu Ile Phe Arg Pro Gly Gly Gly Asp Met Arg Asp Asn Trp Arg Ser Glu Leu Tyr
TCC GAG ATC TTC AGA CCT GGA GGA GGA GAT ATG AGG GAC AAT TGG AGA AGT GAA TTA TAT   7690
III III III III III III III III IIC III III III III III III III III III III III
                                 Gly

490
Lys Tyr Lys Val Val Lys Ile Glu Pro Leu Gly Val Ala Pro Thr Lys Ala Lys Arg Arg
AAA TAT AAA GTA GTA AAA ATT GAA CCA TTA GGA GTA GCA CCC ACC AAG GCA AAG AGA AGA   7750
III III III III III III III III III III III III III III III III III III III III

510     <--- gp120 --------\ /-------- Transmembrane Region (gp 41) ----->
Val Val Gln Arg Glu Lys Arg Ala Val Gly Ile Gly Ala Leu Phe Leu Gly Phe Leu Gly
GTG GTG CAG AGA GAA AAA AGA GCA GTG GGA ATA GGA GCT TTG TTC CTT GGG TTC TTG GGA   7810
III III III III III III III III III III III III III III III III III III III III

530
Ala Ala Gly Ser Thr Met Gly Ala Arg Ser Met Thr Leu Thr Val Gln Ala Arg Gln Leu
GCA GCA GGA AGC ACT ATG GGC GCA CGG TCA ATG ACG CTG ACG GTA CAG GCC AGA CAA TTA   7870
III III III III III III III III GCI III III III III III III III III III III III
                                 Ala

550
Leu Ser Gly Ile Val Gln Gln Gln Asn Asn Leu Leu Arg Ala Ile Glu Ala Gln Gln His
TTG TCT GGT ATA GTG CAG CAG CAG AAC AAT TTG CTG AGG GCT ATT GAG GCG CAA CAG CAT   7930
III III IAI III III III III III III III III III III III III III(---)III III III
        Asp

570
Leu Leu Gln Leu Thr Val Trp Gly Ile Lys Gln Leu Gln Ala Arg Ile Leu Ala Val Glu
CTG TTG CAA CTC ACA GTC TGG GGC ATC AAG CAG CTC CAG GCA AGA ATC·CTG GCT GTG GAA   7990
III III III TCT III III III III III III IIA IIA III III III III III III III III
            Ser                          Lys Gln

590
Arg Tyr Leu Lys Asp Gln Gln Leu Leu Gly Ile Trp Gly Cys Ser Gly Lys Leu Ile Cys
AGA TAC CTA AAG GAT CAA CAG CTC CTG GGG ATT TGG GGT TGC TCT GGA AAA CTC ATT TGC   8050
III III III III III III III III III III III III III III III III III III III III
```

FIG. 5d

```
610                        ▼                      ▼
Thr Thr Ala Val Pro Trp Asn Ala Ser Trp Ser Asn Lys Ser Leu Glu Gln Ile Trp Asn
ACC ACT GCT GTG CCT TGG AAT GCT AGT TGG AGT AAT AAA TCT CTG GAA CAG ATT TGG AAT    8110
||| ||| ||| ||| ||| ||| ||| ||| ||| ||| ||| ||| ||| ||| ||| ||| ||| ||| ||| |||

▼                                               ▼
Asn Met Thr Trp Met Glu Trp Asp Arg Glu Ile Asn Asn Tyr Thr Ser Leu Ile His Ser
AAC ATG ACC TGG ATG GAG TGG GAC AGA GAA ATT AAC AAT TAC ACA AGC TTA ATA CAT TCC    8170
||| ||| ||| ||| ||| ||| ||| ||| ||| ||| ||| ||| ||| ||| ||| ||| ||| ||| ||C |||
                                                                            His
650
Leu Ile Glu Glu Ser Gln Asn Gln Gln Glu Lys Asn Glu Gln Glu Leu Leu Glu Leu Asp
TTA ATT GAA GAA TCG CAA AAC CAG CAA GAA AAG AAT GAA CAA GAA TTA TTG GAA TTA GAT    8230
||| ||| ||| ||| ||| ||| ||| ||| ||| ||| ||| ||| ||| ||| ||| ||| ||| ||| ||| |||

670                                    ▼
Lys Trp Ala Ser Leu Trp Asn Trp Phe Asn Ile Thr Asn Trp Leu Trp Tyr Ile Lys Ile
AAA TGG GCA AGT TTG TGG AAT TGG TTT AAC ATA ACA AAT TGG CTG TGG TAT ATA AAA ATA    8290
||| ||| ||| ||| ||| ||| ||| ||| ||| ||| ||| ||| ||| ||| ||| ||| ||| ||| ||| T||
                                                                               Leu
690
Phe Ile Met Ile Val Gly Gly Leu Val Gly Leu Arg Ile Val Phe Ala Val Leu Ser Ile
TTC ATA ATG ATA GTA GGA GGC TTG GTA GGT TTA AGA ATA GTT TTT GCT GTA CTT TCT ATA    8350
||| ||| ||| ||| ||| ||| ||| ||| ||| ||| ||| ||| ||| ||| ||| ||| ||| ||| ||| |||

710
Val Asn Arg Val Arg Gln Gly Tyr Ser Pro Leu Ser Phe Gln Thr His Leu Pro Thr Pro
GTG AAT AGA GTT AGG CAG GGA TAT TCA CCA TTA TCG TTT CAG ACC CAC CTC CCA ACC CCG    8410
||| ||| ||| ||| ||| ||| ||| ||| ||| ||| ||| ||| ||| ||| ||| ||| ||| ||| |T| |||
                                                                            Ile
730
Arg Gly Pro Asp Arg Pro Glu Gly Ile Glu Glu Glu Gly Gly Glu Arg Asp Arg Asp Arg
AGG GGA CCC GAC AGG CCC GAA GGA ATA GAA GAA GAA GGT GGA GAG AGA GAC AGA GAC AGA    8470
||| ||| ||| ||| ||| ||| ||| ||| ||| ||| ||| ||| ||| ||| ||| ||| ||| ||| ||| |||

750                    ▼
Ser Ile Arg Leu Val Asn Gly Ser END
TCC ATT CGA TTA GTG AAC GGA TCT TAA TTA ATT AA
||| ||| ||| ||| ||| ||| ||| ||| ||| ||| ||| ||
```

FIG. 6

Human HIV-1 Positive Sera

Region of the HIV-1 Envelope

Immunized Rhesus Monkeys

Region of the HIV-1 Envelope